# EUROPEAN PATENT SPECIFICATION

(11) **EP 3 368 013 B1**
(45) Date of publication and mention of the grant of the patent: **08.11.2023**
(21) Application number: 16860815.6
(22) Date of filing: 27.10.2016
(51) Int. Cl.: A61K 31/485, A61K 9/12, A61K 47/10, A61P 25/04

(54) **LIQUID BUPRENORPHINE FORMULATIONS**
FLÜSSIGE BUPRENORPHINFORMULIERUNGEN
FORMULATIONS LIQUIDES DE BUPRÉNORPHINE

(30) Priority: 27.10.2015 US 201514923630
(43) Date of publication of application: 05.09.2018
(73) Proprietor: Benuvia Therapeutics, LLC, Chandler, AZ 85224 (US)
(72) Inventor: AMANCHA, Kiran, P., Chandler, AZ 85286 (US); CHILAMPALLI, Chandeshwari, S., Chandler, AZ 85286 (US); GOSKONDA, Venkat, R., Chandler, AZ 85286 (US)
(74) Representative: Elkington and Fife LLP
(86) International application number: PCT/US2016/059186
(87) International publication number: WO 2017/075256

(56) References cited:
- WO-A1-2008/047163
- WO-A1-2009/127878
- WO-A1-2015/038327
- WO-A1-2015/038327
- FIONA MCINNES ET AL: "Evaluation of the Clearance of a Sublingual Buprenorphine Spray in the Beagle Dog Using Gamma Scintigraphy", PHARMACEUTICAL RESEARCH, KLUWER ACADEMIC PUBLISHERS-PLENUM PUBLISHERS, NL, vol. 25, no. 4, 1 September 2007 (2007-09-01), pages 869-874, XP019613037, ISSN: 1573-904X
- KARI K: "Sublingual buprenorphine vs. morphine for acute pain", AMERICAN FAMILY PHYSICIAN 2012 AMERICAN ACADEMY OF FAMILY PHYSICIANS USA, vol. 86, no. 7, 2012, page 682, ISSN: 0002-838X
- DATABASE EMBASE [Online] ELSEVIER SCIENCE PUBLISHERS, AMSTERDAM, NL; 2012, KARI K: "Sublingual buprenorphine vs. morphine for acute pain", Database accession no. EMB-2012594672 & KARI K: "Sublingual buprenorphine vs. morphine for acute pain", AMERICAN FAMILY PHYSICIAN 2012 AMERICAN ACADEMY OF FAMILY PHYSICIANS USA, vol. 86, no. 7, 2012, page 682, ISSN: 0002-838X

## Description

### Field of the Invention

The invention is directed to liquid formulations containing buprenorphine, a pharmaceutically acceptable salt thereof, or a derivative thereof. The invention is further directed to liquid formulations containing buprenorphine and naloxone, pharmaceutically acceptable salts thereof or derivatives thereof. The invention is further directed to a method of treating pain or opioid dependence by administering liquid formulations containing buprenorphine or buprenorphine and naloxone, pharmaceutically acceptable salts thereof, or derivatives thereof to a patient in need thereof.

### Background of the Invention

Buprenorphine is a semi-synthetic opioid and a partial µ-opioid receptor agonist and has the following structure:

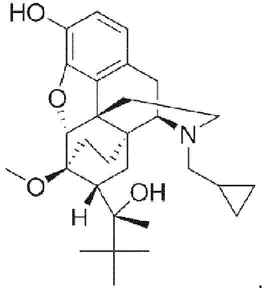

Activation of the µ-opioid receptor leads to antinociception and is the pathway by which opioids such as morphine and fentanyl reduce acute and chronic pain. Buprenorphine has advantages over other opioids such as morphine and fentanyl in that it is only a partial instead of a full agonist of the opioid receptor-like receptor 1 ("ORL1"). Activation of ORL1 has been reported to weaken the analgesic effect induced by the activation of the µ-opioid receptor. Additionally, buprenorphine is an antagonist of δ- and κ-opioid receptors, whose activation has anti-analgesic and psychotomimetic effects, respectively. Buprenorphine is also useful in the management of opioid dependence. The slow binding of buprenorphine to the µ-opioid receptor along with its strong affinity allows for pain management at relatively low blood concentrations and the slow disassociation of buprenorphine from the µ-opioid receptor results in a lack of withdrawal symptoms. WO 2015/038327 A1 discloses buprenorphine sublingual sprays and further discloses methods of treating pain or opioid dependence. WO 2008/047163 A1 and WO 2009/127878 A1 also disclose spray formulations comprising buprenorphine for treating pain. McInnes et al. 2007 discloses the "Evaluation of the Clearance of a Sublingual Buprenorphine Spray in the Beagle Dog Using Gamma Scintigraphy*".*

Buprenorphine is currently available in transdermal patches, intravenous injection, tablet and film strip formulations. Commercially available buprenorphine formulations include Butrans^{®} (Butrans is a registered trademark of Purdue Pharma L.P.), a 7 day transdermal patch that releases buprenorphine at 5, 10 or 20 mcg/hr, and Temgesic, a 0.2 mg sublingual tablet, are used for the treatment of chronic pain. Buprenex^{®} (Buprenex is a registered trademark of Reckitt Benckiser Healthcare (UK) Limited) is a 0.3 mg/mL injectable solution used for the treatment of acute pain. Subutex^{®} (Subutex is a registered trademark of Reckitt Benckiser Healthcare (UK) Limited) and Suboxone^{®} (Suboxone is a registered trademark of Reckitt Benckiser Healthcare (UK) Limited) are tablets used in the treatment of opioid dependence. Subutex^{®} is available in 2 mg and 8 mg sublingual doses of buprenorphine. Suboxone^{®} contains both buprenorphine and naloxone in a 4:1 ratio. Suboxone^{®} is available in tablet form in 2 mg and 8 mg doses. Suboxone^{®} is also available in a sublingual film strip formulation that dissolves faster and is not lost by accidental swallowing.

Naloxone has the following structure and is synthesized from thebaine:

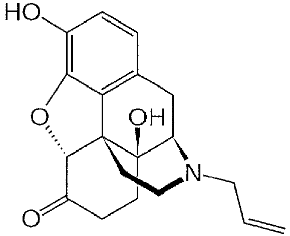

Naloxone is most commonly used to treat patients suffering from opioid dependence or overdose because it is a competitive µ-opioid antagonist that blocks the effects of opioids.

While there are various formulations currently available, there exists a need in the art for a liquid (i.e., sublingual or intranasal) spray formulation containing buprenorphine or buprenorphine and naloxone, pharmaceutically acceptable salts thereof, or derivatives thereof. Such a formulation should be safe, be easy to administer, have a high bioavailability, and be storage stable.

### Summary of the Invention

The present invention is directed to a liquid formulation for use in treating acute pain wherein the formulation is a sublingual spray formulation comprising:
buprenorphine, or a pharmaceutically acceptable salt thereof, in an amount from 0.1625% to 0.65% w/w;
water as a solvent in an amount from 38% to 40% w/w;
a cosolvent consisting of a mixture of ethanol in an amount of 55% w/w and propylene glycol in an amount of about 5% w/w;
an antioxidant consisting of a mixture of butylated hydroxyanisole (BHA) in an amount of about 0.01% w/w and butylated hydroxytoluene (BHT) in an amount of about 0.005% w/w; and
menthol in an amount of about 0.05% w/w, wherein the % w/w is of the total formulation.

In one embodiment, the present invention is directed to a liquid formulation comprising an effective amount of buprenorphine, or a pharmaceutically acceptable salt thereof, wherein the formulation has a pH from about 3.5 to about 5.5.

When the application describes the amounts of buprenorphine and naloxone, all the amounts refer to buprenorphine base and naloxone base, respectively, unless otherwise indicated.

The liquid formulations are the liquid spray formulations.

In an embodiment, the present invention is directed to sublingual spray formulations wherein the Cₘₐₓ (ng/mL) of buprenorphine is from about 0.6 to about 1.5. In one preferred embodiment, the Cₘₐₓ (ng/mL) of buprenorphine is 0.76 following sublingual administration. In another preferred embodiment, the Cₘₐₓ (ng/mL) of buprenorphine is 1.38 following sublingual administration.

In yet another embodiment, the present invention is directed to sublingual spray formulations wherein the Tₘₐₓ of buprenorphine is from about 1.5 to about 1.9 hours. In a preferred embodiment, the Tₘₐₓ of buprenorphine is about 1.75 hours following sublingual administration.

In yet another embodiment, the present invention is directed to sublingual spray formulations wherein the Cₘₐₓ (ng/mL) of buprenorphine is from about 1.2 to about 1.5. In a preferred embodiment, the Cₘₐₓ (ng/mL) of buprenorphine is about 1.38 following sublingual administration.

In a further embodiment, the present invention is directed to sublingual spray formulations wherein the Tₘₐₓ of buprenorphine is from about 1.2 to about 1.7 hours. In a preferred embodiment, the Tₘₐₓ of buprenorphine is about 1.5 hours following sublingual administration.

In a further embodiment, the present invention is directed to sublingual spray formulations wherein the AUC_{0-T} (ng·h/mL) of buprenorphine is from about 2 to about 6 for 0.5 mg dose, and from about 7 to about 11 for 1 mg dose.

In a further embodiment, the present invention is directed to sublingual spray formulations wherein the AUC_{0-∞} (ng·h/mL) of buprenorphine is from about 2 to about 6 for 0.5 mg dose, and from about 7 to about 11 for 1 mg dose.

In another embodiment, the present invention is directed to sublingual spray formulations wherein greater than 98% of the formulation particles are greater than 10 microns in diameter during administration.

In another embodiment, the present invention is directed to sublingual spray formulations wherein the mean Dv(10) is from about 10 to about 40 microns during administration.

In another embodiment, the present invention is directed to sublingual spray formulations wherein the mean Dv(50) is from about 30 to about 80 microns during administration.

In another embodiment, the present invention is directed to sublingual spray formulations wherein the mean Dv(90) is from about 80 to about 200 microns during administration.

In a further embodiment, the present invention is directed to sublingual spray formulations that when administered provide a spray plume ovality ratio of from about 1.1 to 2.4.

In yet another embodiment, the invention is directed to sublingual formulations that when administered provide a plume width of from about 25 to about 45 millimeters.

In a further embodiment, the invention is directed to sublingual formulations that when administered provide a plume angle of from about 30 to about 55 degrees.

In yet another embodiment, the invention is directed to sublingual formulations that when administered provide a D(4,3) of 55 to 95 microns.

In an additional embodiment, the invention is directed to sublingual formulations that when administered provide a spray span ((Dv90-Dv10)/Dv50) of from about 1.2 to about 3.3.

### Brief Description of the Drawings

The patent or application file contains at least one drawing executed in color. Copies of this patent or patent application publication with color drawing(s) will be provided by the Office upon request and payment of the necessary fee.
Fig. 1 depicts a flow chart describing the disposition of the study of the effect of buprenorphine sublingual spray to treat bunionectomy-related pain.
Fig. 2 depicts a chart of a chart of Numeric Rating Scale (NRS) Summed Pain Intensity Difference (SPID) at 4, 8, 24 and 48 hours.
Fig. 3 depicts a chart of time of onset of analgesia for placebo, 0.5 mg tid, 0.25 mg tid and 0.125 tid doses.

### Detailed Description of the Invention

The present invention is directed to a liquid formulation for use in treating acute pain, wherein the formulation is a sublingual spray formulation comprising an effective amount of buprenorphine, or pharmaceutically acceptable salts thereof.

Applicants developed new liquid buprenorphine formulations that unexpectedly are storage stable, safe and effective. Specifically, Applicants were surprised that the formulations were stable at high temperatures (40 degrees Celsius) for an extended period of time (see Examples 1 and 2 below). Further, Applicants unexpectedly found that the formulations provided a quick onset of action and bioavailability (as demonstrated by pharmacokinetic studies, see Example 3 below). The formulations upon administration exhibit excellent droplet size distribution, as well.

As used herein the term "patient" refers but is not limited to a person that is being treated for pain, opioid dependence or another affliction or disease that can be treated with buprenorphine.

As used herein the term "pharmaceutically acceptable" refers to ingredients that are not biologically or otherwise undesirable in a sublingual dosage form.

As used herein the term "effective amount" refers to the amount necessary to treat a patient in need thereof.

As used herein the term "liquid" refers to a sublingual, intranasal or otherwise administered through a mouth or a nose formulation.

As used herein the term "sublingual" refers to administration of a substance via the mouth in such a way that the substance is rapidly absorbed via the blood vessels under the tongue.

As used herein the term "intranasal" refers to administration of the composition to any portion of the nasal epithelium.

Pharmaceutically acceptable salts that can be used in accordance with the current invention include but are not limited to hydrochloride, hydrobromide, hydroiodide, nitrate, sulfate, bisulfate, phosphate, acid phosphate, isonicotinate, acetate, lactate, salicylate, citrate, tartrate, pantothenate, bitartrate, ascorbate, succinate, maleate, gentisinate, fumarate, gluconate, glucaronate, saccharate, formate, benzoate, glutamate, methanesulfonate, ethanesulfonate, benzensulfonate, p-toluenesulfonate and pamoate (i.e., 1,1'-methylene-bis-(2-hydroxy-3-naphthoate)) salts.

In preferred embodiments the pharmaceutically acceptable salt is hydrochloride.

Derivatives of buprenorphine are not limited norbuprenorphine, thenorphine, demethoxybuprenorphine and esters and diastereomers of buprenorphine.

The solvent used with the present invention is United States Pharmacopeia ("USP") purified water.

Cosolvents used in accordance with the current invention are a mixture of alcohols and glycols.

Alcohols used in accordance with the current invention are ethanol.

Glycols used in accordance with the current invention are propylene glycol.

The cosolvent is a mixture of propylene glycol at about 5% w/w and ethanol at about 55% w/w.

Solubilizers that can be used in accordance with the current invention are hydroxpropyl beta-cyclodextrin ("HPβCD") and sulfobutylether cyclodextrin or a mixture thereof.

In preferred embodiments the solubilizer is HPβCD.

In more preferred embodiments the amount of HPβCD is from about 10% w/w to 40% w/w. In a most preferred embodiment the amount of HPβCD is about 30% w/w.

Antioxidants used in accordance with the current invention include but are not limited to butylated hydroxyanisole ("BHA"), butylated hydroxytoluene ("BHT"), or a mixture thereof.

The antioxidant is a mixture of about 0.01% w/w of BHA and about 0.005% w/w of BHT.

Permeation enhancers used in accordance with the current invention include but are not limited to menthol.

The amount of permeation enhancer is about 0.05% w/w of menthol.

Chelating agents that can be used in accordance with the present invention include but are not limited to ethylenediaminetetraacetic acid disodium ("disodium edetate" or edetate disodium dihydrate").

In preferred embodiments the amount of disodium edetate is about 0.005% to about 0.01% w/w.

Formulations of the present invention may have a pH range from about 3.0 to about 7.0, preferably from about 3.5 to about 5.5 and more preferably from about 3.8 to about 5.1. pH adjustors that can be used in accordance with the present invention include but are not limited to citric acid, sodium hydroxide and a mixture thereof. In preferred embodiments the amount of citric acid is from about 2% to about 20% w/w. In more preferred embodiments the amount of citric acid is about 15%. In other more preferred embodiments the amount of citric acid is about 10%.

As used herein, all numerical values relating to amounts, weights, and the like, that are defined as "about" each particular value is plus or minus 10%. For example, the phrase "about 10% w/w" is to be understood as "9% to 11% w/w." Therefore, amounts within 10% of the claimed value are encompassed by the scope of the claims.

As used herein "% w/w" refers to the percent weight of the total formulation.

### Representative Embodiments

In one embodiment, the present invention is directed to a sublingual spray formulation comprising:
an amount of buprenorphine from about 0.1625% to about 0.65% w/w;
an amount of water from about 38% to about 40% w/w;
a cosolvent consisting of a mixture of ethanol in an amount of 55% w/w and propylene glycol in an amount of about 5% w/w;
an antioxidant consisting of a mixture of butylated hydroxyanisole (BHA)
in an amount of about 0.01% w/w and butylated hydroxytoluene (BHT) in an amount of about 0.005% w/w; and
menthol in an amount of about 0.05% w/w.

In one embodiment, the present invention is directed to a sublingual spray formulation comprising:
buprenorphine, or a pharmaceutically acceptable salt thereof in an amount
from about 0.1625% to about 0.65% w/w;
water as a solvent in an amount of from about 38% to about 40% w/w;
a cosolvent consisting of a mixture of ethanol in an amount of 55% w/w and propylene glycol in an amount of about 5% w/w;
the antioxidant consisting of a mixture of butylated hydroxyanisole (BHA) in an amount of about 0.01% w/w and butylated hydroxytoluene (BHT) in an amount of about 0.005% w/w; and
menthol at an amount of about 0.05% w/w;
wherein the % w/w is of the total formulation.

In one embodiment, the sublingual spray formulation comprises:
an amount of buprenorphine from about 0.813% to about 1.3% w/w, preferably 0.0813% w/w, 0.1625% w/w, 0.325% w/w, 0.65% w/w or 1.3% w/w;
an amount of BHA of about 0.01% w/w;
an amount of BHT of about 0.005% w/w;
an amount of ethanol of about 55% w/w;
an amount of propylene glycol of about 5% w/w; and
an amount of water from about 39.8537% to about 38.635% w/w, preferably 39.8537% w/w, 39.7725% w/w, 39.61% w/w, 39.285% w/w or 38.635% w/w.

The following examples are intended to illustrate the present invention and to teach one of ordinary skill in the art how to make and use the invention. They are not intended to be limiting in any way.

### Examples

### Example 1: Stable Buprenorphine Formulations

### Method of Making the Formulations

Sublingual spray formulations were created by first degassing ethanol and USP purified water, separately. Next, the ethanol and purified water were each purged with nitrogen. Soluble excipients were then dissolved in either the ethanol or the purified water based on their solubility. Next, the solutions were combined. Active pharmaceutical ingredient/s was/were added to the final solution and mixed until dissolved.

### Formulations

**Table 1. Stable Sublingual Buprenorphine Spray Formulations**

| **Formulation** | Control | #1 | #2 | #3 | #4 | #5 | #6 | #7 | #8 | #9 |
|---|---|---|---|---|---|---|---|---|---|---|
| Buprenorphine HCl | 0.538 | 0.538 | 0.538 | 0.538 | 0.538 | 0.538 | 0.538 | 0.538 | 0.538 | 0.538 |
| Water (USP) | 39.462 | 39.452 | 39.397 | 39.372 | 89.427 | 94.427 | 39.39 | 39.4 | 39.405 | 69.472 |
| Ethanol | | | | | | | 55 | 55 | | |
| Propylene Glycol | | | | | | 5 | 5 | 5 | | |
| HPβCD | | | | | | | | | | |
| BHA | | | | | | | | | | |
| BHT | | | | | | | | | | |
| Sodium Ascorbate | | | | | | 0.02 | 01 | | | |
| Sodium Thiosulfate | | | | | | | | 0.01 | | |
| Methionine | | | | | | | | | | |
| Menthol | | | | | | | 05 | 0.05 | | |
| Citric Acid | | | | | | 015 | | 0.002 | | |
| Disodium Edetate | | | | | | | 01 | | | |
| pH | 5.09 | 4.99 | 5.11 | 4.71 | 4.01 | 4 | 4.43 | 3.9 | 3.85 | No Data |

| | | | | | | | | | | |
|---|---|---|---|---|---|---|---|---|---|---|
| values = % w/w | | | | | | | | | | |

### Stability Data

The formulations listed in Table 1 were subject to stability test at 40° C ± 2°C under 75% ± 5% relative humidity for six months. Stability data was collected at zero, and six months. Assay and impurities were detected using high performance liquid chromatography with an ultraviolet detector. The assay was performed at 288 nm and indicated as a % of initial concentration. For all impurities, analysis was performed at 240 nm and expressed as a % area. Amounts of particular impurities are listed in Table 2 as a percentage of the area of each formulation along with amount of total impurities.

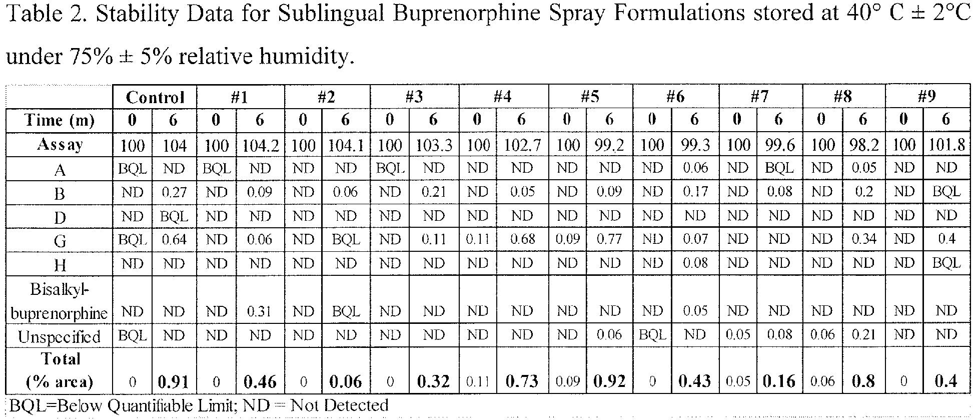

Sublingual buprenorphine spray formulations contained less than one percent total impurities after six months at 40° C. Control and formulations 1, 3, 4, 5, 6, 8 and 9 showed significant increase in levels of individual impurities (impurity B, impurity G, bisalkyl or unspecified impurity) at the 6 month time point whereas formulations containing BHA and BHT (#2; according to the invention) or sodium thiosulfate (#7) showed good stability. pH also played a role in the stability of the product. These results represent sublingual buprenorphine spray formulations that would remain stable for two years at room temperature.

### Example 2: Stable Buprenorphine/Naloxone Formulations

### Method of Making the Formulations

Sublingual spray formulations were created by first degassing ethanol and USP purified water, separately. Next, the ethanol and purified water were each purged with nitrogen. Soluble excipients were then dissolved in either the ethanol or the purified water based on their solubility. Next, the solutions were combined. Buprenorphine and naloxone were added to the final solution and mixed until dissolved.

### Formulations

**Table 3. Stable Buprenorphine/Naloxone Sublingual Spray Formulations**

| **Formulation** | Control #2 | #10 | #11 | #12 | #13 |
|---|---|---|---|---|---|
| Buprenorphine HCl | 8.602 | 8.602 | 8.602 | 8.602 | 8.602 |
| Naloxone HCl | 2.44 | 2.44 | 2.44 | 2.44 | 2.44 |
| Water (USP) | 28.958 | 28.9455 | 28.943 | 28.938 | 28.933 |
| Ethanol | 55 | 55 | 55 | 55 | 55 |
| Propylene Glycol | 5 | 5 | 5 | 5 | 5 |
| BHA | | | | 0.0 | |
| BHT | | | | 0.00 | |
| Sodium Ascorbate | | | | | |
| Sodium Thiosulfate | | 0.0 | | | |
| Citric Acid | | 0.0025 | | | |
| Disodium Edetate | | | 0.005 | 0.005 | 0.005 |

| | | | | | |
|---|---|---|---|---|---|
| values=% w/w | | | | | |

### Stability Data

The formulations listed in Table 3 were subject to stability test at 40° C ± 2°C under 75% ± 5% relative humidity for three months and at ± 25°C under 60% ± 5% relative humidity for three months. Stability data was collected at zero, one, two and three months at 40° C and at zero, one and three months at 25° C. Assay and impurities were detected using high performance liquid chromatography with an ultraviolet detector. Buprenorphine assay was performed at 288 nm and indicated as a % of initial concentration. For all buprenorphine impurities, analysis was performed at 240 nm and expressed as a % area. Naloxone assay was performed at 280 nm and indicated as a % of initial concentration and for all naloxone impurities, analysis was performed at 230 nm. Amounts of particular impurities are listed in Tables 4 and 5 for 40° C and in Table 6 for 25° C as a percentage of the area of each formulation along with amount of total impurities. Relative retention time ("RRT") is given for each impurity.

**Table 4: Stability Data for Control #2 stored at 40° C ± 2°C / 75% ± 5% relative humidity for 1, 2 and 3 months.**

| **40° C** | **Control #2** | | | | | **40° C** | **Control #2** | | | | |
|---|---|---|---|---|---|---|---|---|---|---|---|
| **Buprenorphine** | **RRT** | **0 in** | **1 m** | **2 m** | **3 m** | **Naloxone** | **RRT** | **0 m** | **1 m** | **2 m** | **3 m** |
| Assay | | 100% | 96.93% | 94.22% | 94.27% | Assay | | 100% | 96.31% | 97.22% | 95.62% |
| Impurity B | 0.4 | ND | ND | 0.09% | 0.12% | Impurity C | 0.66 | ND | 1.11% | 1.71% | 2.02% |
| Impurity J | 1.1 | ND | ND | BQL | BQL | Impurity A | 0.83 | ND | ND | 0.10% | 0.19% |
| Impurity F | 1.27 | ND | ND | BQL | BQL | Impurity E | 2.85 | ND | ND | 0.09% | ND |
| Impurity G | 1.8 | 0.11% | 1.84% | 3.10% | 4.14% | Impurity D | 0.20 | ND | ND | ND | 0.09% |
| Unknown Impurities | 0.26 | ND | ND | ND | BQL | Unknown Impurities | 0.28 | ND | 0.09% | 0.17% | 0.23% |
| | 0.86 | ND | 0.28% | 0.46% | 0.63% | | 0.30 | ND | ND | 0.09% | 0.17% |
| | 2.15 | ND | 0.23% | 0.33% | 0.42% | | 0.47 | ND | ND | ND | 0.06% |
| Total (% area) | | 0.11% | 2.35% | 3.98% | 5.31% | | 0.52 | ND | 0.34% | 0.73% | 1.17% |
| BQL = Below Qantifiable Limit; ND = Not Detected | | | | | | | 4.30 | ND | ND | ND | 0.33% |
| | | | | | | Total (% area) | | 0.00% | 1.54% | 2.89% | 4.26% |

The control formulation for the buprenorphine/naloxone sublingual spray formulation contained greater than 1% impurities of both buprenorphine and naloxone within one month at 40° C and between about 4% and about 5% at three months.

**Table 5: Stability Data for Buprenorphine/Naloxone Sublingual Spray Formulations stored at 40° C ± 2°C / 75% ± 5% relative humidity for 1, 2 and 3 months.**

| **40° C** | **#10** | | | | | **#11** | | | | |
|---|---|---|---|---|---|---|---|---|---|---|
| **Buprenorphine** | **RRT** | **0 m** | **1 m** | **2 m** | **3 m** | **RRT** | **0 m** | **1 m** | **2 m** | **3 m** |
| Assay | | 100% | 98.72% | 96.90% | 100.06% | | 100% | 99.26% | 98.91% | 99.96% |
| Impurity G | | | | | | | | | | |
| Total (% area) | | 0.00% | 0.00% | 0.00% | 0.00% | | 0.00% | 0.00% | 0.00% | 0.00% |
| | | | | | | | | | | |

| **Naloxone** | **RRT** | **0 m** | **1 m** | **2 m** | **3 m** | **RRT** | **0 m** | **1 m** | **2m** | **3 m** |
|---|---|---|---|---|---|---|---|---|---|---|
| Assay | | 100% | 99.19% | 102.69% | 102.42% | | 100% | 99.84% | 102.75% | 102.00% |
| Impurity C | | | | | | | | | | |
| Unknown Impurities | | | | | | | | | | |
| | | | | | | | | | | |
| Total (% area) | | 0.00% | 0.00% | 0.00% | 0.00% | | 0.00% | 0.00% | 0.00% | 0.00% |
| | | | | | | | | | | |

| **40° C** | **#12** | | | | | **#13** | | | | |
|---|---|---|---|---|---|---|---|---|---|---|
| **Buprenorphine** | **RRT** | **0 m** | **1 m** | **2 m** | **3 m** | **RRT** | **0 m** | **1 m** | **2 m** | **3 m** |
| Assay | | 100 | 99.50% | 101.44% | 101.22% | | 100% | 99.06% | 100.30% | 99.36% |
| Impurity G | 1.8 | ND | ND | ND | 0.05% | | | | | |
| Total (% area) | | 0.00% | 0.00% | 0.00% | 0.05% | | 0.00% | 0.00% | 0.00% | 0.00% |
| | | | | | | | | | | |

| **Naloxone** | **RRT** | **0 m** | **1 m** | **2 m** | **3 m** | **RRT** | **0 m** | **1 m** | **2 m** | **3 m** |
|---|---|---|---|---|---|---|---|---|---|---|
| Assay | | 100% | 97.91% | 102.36% | 103.11% | | 100% | 101.42% | 102.72% | 103.38% |
| Impurity C | 0.66 | ND | ND | 0.11% | 0.14% | 0.66 | ND | ND | ND | 0.09% |
| Unknown Impurities | 0.52 | ND | ND | 0.07% | 0.12% | 0.52 | ND | ND | BQL | ND |
| | 4.02 | ND | ND | ND | ND | | | | | |
| Total (% area) | | 0.00% | 0.00% | 0.18% | 0.26% | | 0.00% | 0.00% | 0.00% | 0.09% |

| | | | | | | | | | | |
|---|---|---|---|---|---|---|---|---|---|---|
| BQL = Below Qantifiable Limit; ND = Not Detected | | | | | | | | | | |

All formulations had less than 1% total impurities at three months. Similar to the buprenorphine only formulations in Example 1, formulations containing sodium thiosulfate (#10 and #11) were exceptionally stable with no impurities after three months. Formulation #12 (according to the invention) contains BHA and BHT as the antioxidant and had significant impurities of naloxone (0.26% total impurities). Formulation #13 contains sodium ascorbate and had no impurities of buprenorphine and 0.09% total impurities of naloxone. These results represent sublingual spray formulations that would remain stable for one year at room temperature.

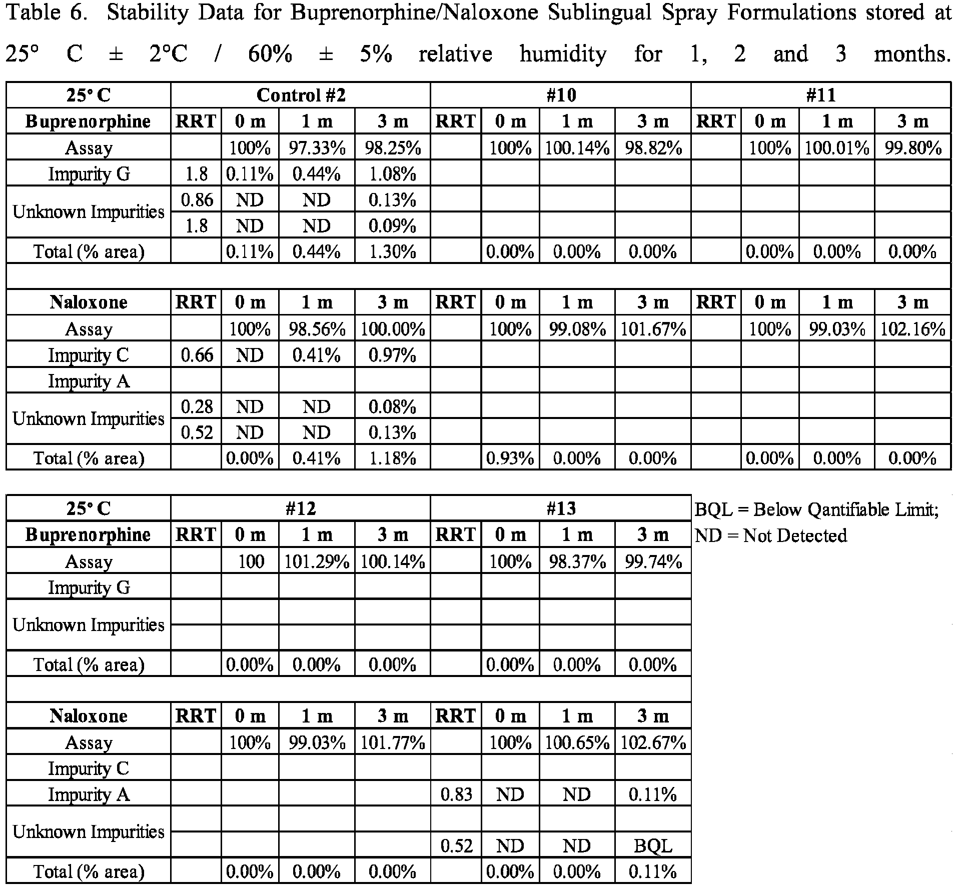

The control formulation had greater than 1% impurities at three months. All formulations containing antioxidants had less than 1% total impurities at three months. Similar to the buprenorphine only formulations in Example 1, formulations containing sodium thiosulfate (#10 and #11) or a mixture of BHA and BHT (#12 according to the invention) were exceptionally stable with no impurities after three months. Formulation #13 which contains sodium ascorbate had no impurities of buprenorphine and 0.11% total impurities of naloxone after storage at 25° C ± 2°C / 75% ± 5% relative humidity.

### Example 3: Pharmacokinetics of Buprenorphine Sublingual Spray Formulations

A study was designed and executed to determine the pharmacokinetics of buprenorphine sublingual spray formulations of the present invention after administration in healthy volunteers under fasting conditions.

The study was a single center, single dose, open-label, 1-sequence, 2-period, ascending dose study design in twelve healthy male and female subjects. The following dose levels of the investigational product were administered under fasting conditions: Dose 1: A single 0.5 mg dose (1 spray of 100 microliters) of Buprenorphine 5 mg/mL Sublingual Spray; and Dose 2: A single 1.0 mg dose (2 sprays of 100 microliters) of Buprenorphine 5 mg/mL Sublingual Spray.

The subjects arrived at the clinical site more than 10 hours before the buprenorphine administration. The subjected were supervised overnight (while fasting) and a single 50 mg dose of naltrexone (1 × 50 mg tablet) was orally administered with 240 mL of water approximately 1 hour prior to the buprenorphine administration to provide blockade of the pharmacological effects of buprenorphine. Then, a single dose (0.5 mg in period 1 and 1.0 mg in period 2) of the buprenorphine formulation was sublingually administered in the morning. Subjects were allowed to leave the clinical site after the 24-hour post-dose blood draw and returned to the clinical site before the remaining blood sample. The second dose level was administered following favorable safety review. The buprenorphine administrations were separated by a wash-out of 14 calendar days. The parameters are summarized below in Table 7.

**Table 7. Summary of Pharmacokinetic Parameters**

| Parameter | Buprenorphine 0.5 mg | | Buprenorphine 1 mg | |
|---|---|---|---|---|
| | MEAN | C.V. | MEAN | C.V. |
| Cₘₐₓ (ng/mL) | 0.761 | 19.0 | 1.38 | 10.2 |
| ln(Cₘₐₓ) | -0.2904 | -67.1 | 0.3169 | 31.2 |
| Tₘₐₓ (hours) * | 1.75 | 30.8 | 1.50 | 30.6 |
| AUC_{0-T} (ng·h/mL) | 4.37 | 13.6 | 9.12 | 10.7 |
| ln(AUC_{0-T}) | 1.4671 | 9.0 | 2.2053 | 5.0 |
| AUC_{0-∞} (ng · h/mL) | 4.81 | 13.3 | 10.2 | 10.6 |
| ln(AUC_{0-∞}) | 1.5614 | 8.7 | 2.3170 | 4.7 |
| AUC_{0-T/∞} (%) | 91.19 | 6.6 | 89.49 | 3.5 |
| λ_{Z} (hours⁻¹) | 0.0959 | 53.3 | 0.0313 | 17.0 |
| T_{half} (hours) | 9.75 | 57.4 | 22.87 | 20.1 |
| V_{D}/F (L) | 1450 | 54.9 | 3250 | 19.4 |
| Cl/F (L/h) | 106 | 13.8 | 99.1 | 11.2 |
| Cₘₐₓ/D (ng/mL) | 0.761 | 19.0 | 0.690 | 10.2 |
| ln(Cₘₐₓ/D) | -0.2904 | -67.1 | -0.3763 | -26.3 |
| AUC_{0-T}/D (ng·h/mL) | 4.37 | 13.6 | 4.56 | 10.7 |
| ln(AUC_{0-T}/D) | 1.4671 | 9.0 | 1.5122 | 7.3 |
| AUC_{0-∞}/D (ng-h/mL) | 4.81 | 13.3 | 5.10 | 10.6 |
| ln(AUC_{0-∞}/D) | 1.5614 | 8.7 | 1.6238 | 6.7 |

| | | | | |
|---|---|---|---|---|
| ^{∗} Tₘₐₓ, the median is presented | | | | |

As seen in Table 7, the Cmax obtained for buprenorphine were 0.761 ng/mL and 1.38 ng/mL. The Tmax observed for buprenorphine was 1.75 and 1.50 hours following the ascending doses.

### Example 4: Bioavailability of Buprenorphine

A study was designed and executed in order to compare the rate and extent of absorption and bioavailability of 1 mg buprenorphine sublingual spray formulations of the present invention with 0.3 mg (1mL) Buprenex^{®} (buprenorphine HCl) intramuscular injection and 0.3 mg (1 mL) Buprenex^{®} (buprenorphine HCl) intravenous bolus injection.

This was an open-label, 3-treatment, 3-period, 6-sequence, single-dose, randomized crossover study. Eighteen healthy male and female volunteers were randomly assigned to 1 of 6 treatment sequences. Dosing occurred after an overnight fast and there was a minimum 14-day washout between the dosing in two periods. Blood samples for the measurement of the plasma concentrations of buprenorphine were collected before (pre-dose) and at 5, 10, 20, 30, and 40 minutes and at 1, 1.25, 1.5, 2, 4, 6, 8, 10, 12, 16, 24, 36, 48, 72, 96, 120, and 144 hours after dosing. The results of this study are summarized below in Table 8.

**Table 8: Bioavailability of Buprenorphine**

| Pa rameter^{∗} | Sublingual 1 mg Spray | Intramuscular 0,3 mg | Intravenous 0.3 mg |
|---|---|---|---|
| Cmax(ng/mL) | 1.20 ± 0.507 (18) | 1.73 ± 1.08 (18) | 3.95 ± 3.66 (18) |
| Tmax(h) | 1.50 (18) | 0.17 (18) | 0.083 (18) |
| | [0.50 - 2.00] | [0.083 - 1.50] | [0.083 - 0.333] |
| AUC(0-t) (h×ng/mL) | 7.31 ± 2.80 (18) | 4.97 ± 0.90 (18) | 5.09 ± 1.01 (18) |
| AUC(inf) (h×ng/mL) | 8.19 ± 3.27 (15) | 5.50 ± 0.83 (15) | 5.51 ± 1.21 (17) |
| λz (1/h) | 0.0551 ± 0.0357 (15) | 0.0655 ± 0.0210 (15) | 0.1028 ± 0.0641 (17) |
| t½ (h) | 17.1 ± 8.62 (15) | 12.0 ± 5.31 (15) | 9.37 ± 6.49 (17) |

The absolute bioavailability of buprenorphine, based on AUC(0-t) and AUC(inf), after sublingual administration was 41.03% and 42.57%, respectively.

### Example 5: Buprenorphine Spray Droplet Size Distribution, Spray Pattern and Plume Geometry

A challenge of creating a buprenorphine sublingual spray formulation is that it must be capable of producing spray droplets that are over 10 microns in diameter. Spray droplets 10 microns or smaller could be inhaled into the lungs. The optimal particle size for sublingual spray droplets is from 20 to about 200 microns in diameter. It is desirable for the formulation to have droplet sizes near 20 because this increases the surface area and increased surface area exposure is one factor that contributes to a high bioavailability. Sublingual formulations should be able to maintain a consistent droplet size throughout its shelf life. Applicants found during testing that formulations of the present invention yielded desirable droplet sizes for sublingual administration. The testing also revealed that the formulation dose remains consistent when administered with a spray pump.

Five milligram per mL buprenorphine spray formulations of the present invention were subjected to two different storage conditions (25 and 40 degrees C) and samples were taken at two different times (5M and 6M) for spray droplet size distribution analysis. Droplet analysis was conducted using standard laser analysis procedures known by those of skill in the art.

Droplet size distribution (Dv10, Dv50, Dv90, percent droplets less than 10 micrometers in diameter, D(4,3) and Span tested at two distances, 3 cm and 6 cm for upright and horizontal samples stored at 25 and 40 degrees C) and spray pattern (Dmin, Dmax and ovality ratio tested at two distances, 3 cm and 6 cm for upright and horizontal samples stored at 25 and 40 degrees C) were determined. D(4,3) refers to the volume moment mean of the particles; Dv10 refers to droplet size for which 10% of the total volume is obtained; Dv50 refers to droplet size for which 50% of the total volume is obtained; Dv90 refers to droplet size for which 90% of the total volume is obtained; Span refers to distribution span (Dv90-Dv10)/Dv50; DSD refers to droplet size distribution; the temperature listed is the storage temperature; U refers to an upright position of the spray pump; and H refers to horizontal position of the spray pump. The results of these studies can be seen below in Tables 9 to 40.

In addition, the formulations were tested for plume geometry including width and angle using standard procedures known by those of skill in the art. This testing showed that the spray pattern and plume were acceptable for formulations of the present invention. The results of these studies can be seen below in Tables 41 and 42.

**Table 9. Droplet Size Distribution at 3 cm for sample stored at 25 degrees C, Upright position, 5M**

| | **DSD 3cm 25°C - U** | **Dv(10) (µm)** | **Dv(50) (µm)** | **Dv(90) (µm)** | **%<10µ** | **D(4,3) (µm)** | **Span** |
|---|---|---|---|---|---|---|---|
| | Mean | 25.37 | 53.25 | 111.1 | 0.9507 | 62.07 | 1.609 |
| Range | Min | 24.38 | 51.44 | 106.0 | 0.8534 | 59.51 | 1.539 |
| | Max | 26.20 | 55.85 | 119.4 | 1.0410 | 65.72 | 1.705 |

**Table 10. Droplet Size Distribution at 6 cm for sample stored at 25 degrees C, Upright position, 5M**

| | **DSD 6cm 25°C - U** | **Dv(10) (µm)** | **Dv(50) (µm)** | **Dv(90) (µm)** | **%<10µ** | **D(4,3) (µm)** | **Span** |
|---|---|---|---|---|---|---|---|
| | Mean | 30.58 | 56.68 | 102.7 | 1.5794 | 62.37 | 1.270 |
| Range | Min | 28.93 | 52.00 | 90.5 | 1.4610 | 56.45 | 1.171 |
| | Max | 31.60 | 60.47 | 113.4 | 1.7840 | 67.41 | 1.355 |

**Table 11. Droplet Size Distribution at 3 cm for sample stored at 25 degrees C, Horizontal position, 5M**

| | **DSD 3cm 25°C - H** | **Dv(10) (µm)** | **Dv(50) (µm)** | **Dv(90) (µm)** | **%<10µ** | **D(4,3) (µm)** | **Span** |
|---|---|---|---|---|---|---|---|
| | Mean | 24.65 | 53.78 | 138.2 | 0.7813 | 72.37 | 2.123 |
| Range | Min | 21.87 | 50.76 | 105.8 | 0.0000 | 59.42 | 1.593 |
| | Max | 26.70 | 58.10 | 194.5 | 1.1560 | 89.39 | 3.295 |

**Table 12. Droplet Size Distribution at 6 cm for sample stored at 25 degrees C, Horizontal position, 5M**

| | **DSD 6cm 25°C - H** | **Dv(10) (µm)** | **Dv(50) (µm)** | **Dv(90) (µm)** | **%<10µ** | **D(4,3) (µm)** | **Span** |
|---|---|---|---|---|---|---|---|
| | Mean | 30.18 | 55.86 | 108.3 | 0.8612 | 68.69 | 1.403 |
| Range | Min | 26.86 | 52.98 | 96.1 | 0.0637 | 63.28 | 1.171 |
| | Max | 32.03 | 59.90 | 124.7 | 1.6630 | 74.75 | 1.782 |

**Table 13. Droplet Size Distribution at 3 cm for sample stored at 40 degrees C, Upright position, 5M**

| | **DSD 3cm 40°C - U** | **Dv(10) (µm)** | **Dv(50) (µm)** | **Dv(90) (µm)** | **%<10µ** | **D(4,3) (µm)** | **Span** |
|---|---|---|---|---|---|---|---|
| | Mean | 26.75 | 56.64 | 120.3 | 0.9120 | 66.53 | 1.651 |
| Range | Min | 26.22 | 55.44 | 116.8 | 0.7907 | 65.09 | 1.612 |
| | Max | 27.33 | 58.02 | 122.7 | 0.9900 | 67.94 | 1.689 |

**Table 14. Droplet Size Distribution at 6 cm for sample stored at 40 degrees C, Upright position, 5M**

| | **DSD 6cm 40°C - U** | **Dv(10) (µm)** | **Dv(50) (µm)** | **Dv(90) (µm)** | **%<10µ** | **D(4,3) (µm)** | **Span** |
|---|---|---|---|---|---|---|---|
| | Mean | 32.87 | 63.39 | 121.7 | 1.3128 | 71.44 | 1.390 |
| Range | Min | 31.62 | 59.93 | 111.7 | 0.6002 | 66.68 | 1.280 |
| | Max | 35.85 | 79.44 | 174.7 | 1.5100 | 94.26 | 1.748 |

**Table 15. Droplet Size Distribution at 3 cm for sample stored at 40 degrees C, Horizontal position, 5M**

| | **DSD 3cm 40°C - H** | **Dv(10) (µm)** | **Dv(50) (µm)** | **Dv(90) (µm)** | **%<10µ** | **D(4,3) (µm)** | **Span** |
|---|---|---|---|---|---|---|---|
| | Mean | 26.08 | 55.51 | 116.1 | 0.8906 | 64.59 | 1.619 |
| Range | Min | 24.86 | 51.65 | 104.2 | 0.7230 | 59.27 | 1.530 |
| | Max | 27.12 | 58.59 | 126.6 | 1.0880 | 69.05 | 1.710 |

**Table 16. Droplet Size Distribution at 6 cm for sample stored at 40 degrees C, Horizontal position, 5M**

| | **DSD 6cm 40°C** - H | **Dv(10) (µm)** | **Dv(50) (µm)** | **Dv(90) (µm)** | **%<10µ** | D(4,3) **(µm)** | Span |
|---|---|---|---|---|---|---|---|
| | Mean | 30.96 | 57.88 | 105.6 | 1.5678 | 63.84 | 1.288 |
| Range | Min | 29.43 | 54.51 | 97.5 | 1.1350 | 59.57 | 1.195 |
| | Max | 31.84 | 62.23 | 120.3 | 1.7230 | 70.09 | 1.429 |

**Table 17. Plume Geometry at 3 cm for sample stored at 40 degrees C, Upright position, 5M**

| | **Spray Pattern 3cm 40°C - U** | **Dmin (mm)** | **Dmax (mm)** | **Ovality Ratio** |
|---|---|---|---|---|
| | Mean | 12.8 | 20.0 | 1.584 |
| Range | Min | 11.6 | 17.2 | 1.289 |
| | Max | 13.6 | 24.7 | 2.043 |

**Table 18. Plume Geometry at 6 cm for sample stored at 25 degrees C, Horizontal position, 5M**

| | **Spray Pattern 6cm 25°C - H** | **Dmin (mm)** | **Dmax (mm)** | **Ovality Ratio** |
|---|---|---|---|---|
| | Mean | 21.4 | 29.1 | 1.362 |
| Range | Min | 20.2 | 27.1 | 1.228 |
| | Max | 22.5 | 32.0 | 1.511 |

**Table 19. Plume Geometry at 3 cm for sample stored at 25 degrees C, Horizontal position, 5M**

| | **Spray Pattern 3cm 25°C** - **H** | **Dmin (mm)** | **Dmax (mm)** | **Ovality Ratio** |
|---|---|---|---|---|
| | Mean | 13.6 | 19.5 | 1.436 |
| Range | Min | 13.0 | 18.0 | 1.382 |
| | Max | 14.2 | 21.1 | 1.580 |

**Table 20. Plume Geometry at 6 cm for sample stored at 25 degrees C, Upright position, 5M**

| | **Spray Pattern 6cm 25°C - U** | **Dmin (mm)** | **Dmax (mm)** | **Ovality Ratio** |
|---|---|---|---|---|
| | Mean | 21.3 | 30.1 | 1.421 |
| **Range** | Min | 19.9 | 26.7 | 1.244 |
| | Max | 22.3 | 33.4 | 1.679 |

**Table 21. Plume Geometry at 3 cm for sample stored at 25 degrees C, Upright position, 5M**

| | **Spray Pattern 3cm 25°C - U** | **Dmin (mm)** | **Dmax (mm)** | **Ovality Ratio** |
|---|---|---|---|---|
| | Mean | 14.4 | 19.1 | 1.320 |
| **Range** | Min | 13.2 | 17.1 | 1.212 |
| | Max | 15.9 | 22.3 | 1.426 |

**Table 22. Plume Geometry at 3 cm for sample stored at 40 degrees C, Horizontal position, 5M**

| | **Spray Pattern 3cm 40°C - H** | **Dmin (mm)** | **Dmax (mm)** | **Ovality Ratio** |
|---|---|---|---|---|
| | Mean | 13.0 | 18.3 | 1.415 |
| **Range** | Min | 12.3 | 16.1 | 1.180 |
| | Max | 13.9 | 21.3 | 1.662 |

**Table 23. Plume Geometry at 6 cm for sample stored at 40 degrees C, Upright position, 5M**

| | **Spray Pattern 6cm 40°C - U** | **Dmin (mm)** | **Dmax (mm)** | **Ovality Ratio** |
|---|---|---|---|---|
| | Mean | 20.8 | 32.2 | 1.578 |
| Range | Min | 18.3 | 25.3 | 1.151 |
| | Max | 22.2 | 43.2 | 2.317 |

**Table 24. Plume Geometry at 6 cm for sample stored at 40 degrees C, Horizontal position, 5M**

| | **Spray Pattern 6cm 40°C - H** | **Dmin (mm)** | **Dmax (mm)** | **Ovality Ratio** |
|---|---|---|---|---|
| | Mean | 21.5 | 29.4 | 1.371 |
| Range | Min | 19.8 | 27.1 | 1.253 |
| | Max | 23.3 | 32.5 | 1.639 |

**Table 25. Droplet Size Distribution at 3 cm for sample stored at 25 degrees C, Upright position, 6M**

| | **DSD 3cm 25°C** - **U** | **Dv(10) (µm)** | **Dv(50) (µm)** | **Dv(90) (µm)** | **%<10µ** | **D(4,3) (µm)** | **Span** |
|---|---|---|---|---|---|---|---|
| | Mean | 26.22 | 57.53 | 121.8 | 0.5523 | 67.25 | 1.652 |
| Range | Min | 24.63 | 50.98 | 104.4 | 0.0000 | 59.18 | 1.544 |
| | Max | 27.73 | 68.01 | 148.6 | 0.9883 | 79.42 | 1.783 |

**Table 26. Droplet Size Distribution at 6 cm for sample stored at 25 degrees C, Upright position, 6M**

| | **DSD 6cm 25°C - U** | **Dv(10) (µm)** | **Dv(50) (µm)** | **Dv(90) (µm)** | **%<10µ** | **D(4,3) (µm)** | **Span** |
|---|---|---|---|---|---|---|---|
| | Mean | 31.87 | 62.59 | 119.9 | 1.1915 | 70.21 | 1.405 |
| Range | Min | 29.24 | 58.74 | 111.6 | 0.8993 | 65.79 | 1.282 |
| | Max | 33.93 | 66.29 | 133.7 | 1.4090 | 75.92 | 1.528 |

**Table 27. Droplet Size Distribution at 3 cm for sample stored at 25 degrees C, Horizontal position, 6M**

| | **DSD 3cm 25°C - H** | **Dv(10) (µm)** | **Dv(50) (µm)** | **Dv(90) (µm)** | **%<10µ** | **D(4,3) (µm)** | **Span** |
|---|---|---|---|---|---|---|---|
| | Mean | 24.55 | 50.03 | 101.6 | 0.8918 | 57.62 | 1.538 |
| Range | Min | 22.88 | 46.53 | 91.7 | 0.0000 | 52.75 | 1.476 |
| | Max | 25.64 | 52.39 | 109.5 | 1.3350 | 61.24 | 1.633 |

**Table 28. Droplet Size Distribution at 6 cm for sample stored at 25 degrees C, Horizontal position, 6M**

| | **DSD 6cm 25°C** - **H** | **Dv(10) (µm)** | **Dv(50) (µm)** | **Dv(90) (µm)** | **%<10µ** | **D(4,3) (µm)** | **Span** |
|---|---|---|---|---|---|---|---|
| | Mean | 29.58 | 55.85 | 105.2 | 1.3818 | 62.82 | 1.323 |
| Range | Min | 28.53 | 51.57 | 89.4 | 1.0870 | 55.73 | 1.178 |
| | Max | 30.75 | 60.69 | 116.4 | 1.6780 | 67.86 | 1.434 |

**Table 29. Droplet Size Distribution at 3 cm for sample stored at 40 degrees C, Upright position, 6M**

| | **DSD 3cm 40°C** - **U** | **Dv(10) (µm)** | **Dv(50) (µm)** | **Dv(90) (µm)** | **%<10µ** | **D(4,3) (µm)** | **Span** |
|---|---|---|---|---|---|---|---|
| | Mean | 27.60 | 58.79 | 125.9 | 0.4862 | 69.31 | 1.669 |
| Range | Min | 26.50 | 52.85 | 111.3 | 0.0000 | 62.36 | 1.579 |
| | Max | 29.11 | 65.51 | 140.0 | 0.7686 | 76.44 | 1.729 |

**Table 30. Droplet Size Distribution at 6 cm for sample stored at 40 degrees C, Upright position, 6M**

| | **DSD 6cm 40°C - U** | **Dv(10) (µm)** | **Dv(50) (µm)** | **Dv(90) (µm)** | **%**<10µ | **D(4,3) (µm)** | **Span** |
|---|---|---|---|---|---|---|---|
| | Mean | 33.68 | 67.20 | 131.3 | 1.0200 | 76.03 | 1.450 |
| Range | Min | 32.54 | 63.80 | 118.0 | 0.8835 | 70.69 | 1.314 |
| | Max | 35.01 | 70.75 | 141.2 | 1.4480 | 80.26 | 1.543 |

**Table 31. Droplet Size Distribution at 3 cm for sample stored at 40 degrees C, Horizontal position, 6M**

| | **DSD 3cm 40°C** - **H** | **Dv(10) (µm)** | **Dv(50) (µm)** | **Dv(90) (µm)** | **%<10µ** | **D(4,3) (µm)** | **Span** |
|---|---|---|---|---|---|---|---|
| | Mean | 27.75 | 55.42 | 114.3 | 0.0005 | 64.60 | 1.559 |
| Range | Min | 26.47 | 52.01 | 104.6 | 0.0000 | 60.13 | 1.475 |
| | Max | 29.22 | 59.01 | 124.9 | 0.0019 | 69.62 | 1.621 |

**Table 32. Droplet Size Distribution at 6 cm for sample stored at 40 degrees C, Horizontal position, 6M**

| | **DSD 6cm 40°C - H** | **Dv(10) (µm)** | **Dv(50) (µm)** | **Dv(90) (µm)** | **%<10µ** | **D(4,3) (µm)** | **Span** |
|---|---|---|---|---|---|---|---|
| | Mean | 34.33 | 63.86 | 118.0 | 0.9685 | 70.95 | 1.309 |
| Range | Min | 32.47 | 60.19 | 110.1 | 0.0624 | 66.54 | 1.251 |
| | Max | 37.21 | 68.17 | 129.6 | 1.5090 | 76.88 | 1.363 |

**Table 33. Plume Geometry at 3 cm for sample stored at 25 degrees C, Upright position, 6M**

| | **Spray Pattern 3cm 25°C** - **U** | **Dmin (mm)** | **Dmax (mm)** | **Ovality Ratio** |
|---|---|---|---|---|
| | Mean | 14.0 | 20.8 | 1.489 |
| Range | Min | 13.4 | 17.9 | 1.300 |
| | Max | 14.5 | 23.1 | 1.664 |

**Table 34. Plume Geometry at 6 cm for sample stored at 25 degrees C, Upright position, 6M**

| | **Spray Pattern 6cm 25°C - U** | **Dmin (mm)** | **Dmax (mm)** | **Ovality Ratio** |
|---|---|---|---|---|
| | Mean | 20.3 | 30.3 | 1.497 |
| Range | Min | 19.1 | 27.4 | 1.320 |
| | Max | 21.1 | 33.6 | 1.705 |

**Table 35. Plume Geometry at 3 cm for sample stored at 25 degrees C, Horizontal position, 6M**

| | **Spray Pattern 3cm 25°C - H** | **Dmin (mm)** | **Dmax (mm)** | **Ovality Ratio** |
|---|---|---|---|---|
| | Mean | 14.0 | 21.4 | 1.549 |
| Range | Min | 12.9 | 19.8 | 1.276 |
| | Max | 15.7 | 23.9 | 1.852 |

**Table 36. Plume Geometry at 6 cm for sample stored at 25 degrees C, Horizontal position, 6M**

| | **Spray Pattern 6cm 25°C - H** | **Dmin (mm)** | **Dmax (mm)** | **Ovality Ratio** |
|---|---|---|---|---|
| | Mean | 20.2 | 32.3 | 1.599 |
| Range | Min | 18.8 | 28.4 | 1.390 |
| | Max | 21.3 | 37.7 | 1.808 |

**Table 37. Plume Geometry at 3 cm for sample stored at 40 degrees C, Upright position, 6M**

| | **Spray Pattern 3cm 40°C** - **U** | **Dmin (mm)** | **Dmax (mm)** | **Ovality Ratio** |
|---|---|---|---|---|
| | Mean | 14.9 | 19.2 | 1.284 |
| Range | Min | 13.8 | 17.3 | 1.155 |
| | Max | 15.5 | 20.8 | 1.399 |

**Table 38. Plume Geometry at 6 cm for sample stored at 40 degrees C, Upright position, 6M**

| | **Spray Pattern 6cm 40°C - U** | **Dmin (mm)** | **Dmax (mm)** | **Ovality Ratio** |
|---|---|---|---|---|
| | Mean | 21.3 | 27.5 | 1.296 |
| Range | Min | 19.8 | 26.5 | 1.194 |
| | Max | 22.8 | 29.3 | 1.427 |

**Table 39. Plume Geometry at 3 cm for sample stored at 40 degrees C, Horizontal position, 6M**

| | **Spray Pattern 3cm 40°C - H** | **Dmin (mm)** | **Dmax (mm)** | **Ovality Ratio** |
|---|---|---|---|---|
| | Mean | **14.6** | 22.5 | **1.547** |
| Range | Min | 13.9 | 20.8 | 1.430 |
| | Max | 16.0 | 24.8 | 1.781 |

**Table 40. Plume Geometry at 6 cm for sample stored at 40 degrees C, Horizontal position, 6M**

| | **Spray Pattern 6cm 40°C - H** | **Dmin (mm)** | **Dmax (mm)** | **Ovality Ratio** |
|---|---|---|---|---|
| | Mean | 21.5 | 29.4 | 1.371 |
| Range | Min | 19.8 | 27.1 | 1.253 |
| | Max | 23.3 | 32.5 | 1.639 |

**Table 41: Plume Geometry at 3 cm (width and angle)**

| | **3cm** | **Width (mm)** | **Angle (°)** |
|---|---|---|---|
| | Mean | 27.9 | 49.9 |
| Range | Min | 25.5 | 46.1 |
| | Max | 30.8 | 54.3 |

**Table 42: Plume Geometry at 6 cm (width and angle)**

| | **6cm** | **Width (mm)** | **Angle (°)** |
|---|---|---|---|
| | Mean | 40.2 | 37.0 |
| Range | Min | 36.0 | 33.4 |
| | Max | 43.9 | 40.2 |

### Example 6. Further Buprenorphine Formulations

### Table 43. Further Buprenorphine Formulations

Formulations #15, #16 and #17 are used in the clinical trial listed as Example 8 for acute pain indication, whereas formulations #14, #15, #16, #17 and #18 will be used in chronic pain indication.

Formulations #14, #15, #16, #17 and #18 represent 0.0625 mg, 0.125 mg, 0.25 mg, 0.5 mg and 1 mg doses, respectively. (Equivalent to buprenorphine base).

| **Formulation** | **#14** | **#15** | **#16** | **#17** | **#18** |
|---|---|---|---|---|---|
| Buprenorphine HCl | 0.0813 | 0.1625 | 0.325 | 0.65 | 1.3 |
| BHA | 0.01 | 0.01 | 0.01 | 0.01 | 0.01 |
| BHT | 0.005 | 0.005 | 0.005 | 0.005 | 0.005 |
| L-Menthol | 0.05 | 0.05 | 0.05 | 0.05 | 0.05 |
| Ethanol | 55 | 55 | 55 | 55 | 55 |
| Propylene Glycol | 5 | 5 | 5 | 5 | 5 |
| Purified Water | 39.8537 | 39.7725 | 39.61 | 39.285 | 38.635 |
| Citric Acid Anhydrous | QS to pH | QS to pH | QS to pH | QS to pH | QS to pH |
| Sodium Hydroxide | QS to pH | QS to pH | QS to pH | QS to pH | QS to pH |
| Nitrogen | Sparging /Overlay | Sparging /Overlay | Sparging /Overlay | Sparging /Overlay | Sparging /Overlay |

| | | | | | |
|---|---|---|---|---|---|
| Values = % w/w. | | | | | |

Buprenorphine formulations of Table 43 were all stable upon preparation.

### Example 7. Further Buprenorphine/Naloxone Formulations

**Table 44. Further Buprenorphine/Naloxone Formulations**

| **Formulation** | **#19** | **#20** | **#21** | **#22** | **#23** | **#24** | **#25** |
|---|---|---|---|---|---|---|---|
| Buprenorphine HCl | 8.39 | 7.68 | 2.84 | 1.42 | 5.70 | 3.75 | 1.04 |
| NaloxoneHCl Dihydrate | 2.37 | 2.19 | 0.80 | 0.40 | 1.61 | 1.06 | 0.29 |
| L-Menthol | 0.05 | 0.05 | 0.05 | 0.05 | 0.05 | 0.05 | 0.05 |
| Edetate Disodium Dihydrate | 0.005 | 0.005 | 0.005 | 0.005 | 0.005 | 0.005 | 0.005 |
| Sodium Ascorbate | 0.02 | 0.02 | 0.02 | 0.02 | 0.02 | 0.02 | 0.02 |
| Ethanol | 55 | 55 | 55 | 55 | 55 | 55 | 55 |
| Propylene Glycol | 5 | 5 | 5 | 5 | 5 | 5 | 5 |
| Water | 29.165 | 30.059 | 36.281 | 38.103 | 32.614 | 35.114 | 38.596 |

| | | | | | | | |
|---|---|---|---|---|---|---|---|
| Values = % w/w. | | | | | | | |

Buprenorphine/naloxone formulations of Table 44 were all stable upon preparation.

### Example 8: Method of Treatment of Pain Using Buprenorphine

### Specifications of the Study

This was a multicenter, randomized, double-blind, multiple-dose, placebo-controlled study evaluating the efficacy and safety of three dosing regimens of Buprenorphine Sublingual Spray (0.5 mg (formulation #17) three times daily ("tid"), 0.25 mg (formulation #16) tid, or 0.125 mg (formulation #15) tid), and/or matching placebo in subjects with moderate to severe postoperative pain after bunionectomy. 322 subjects were randomized. 298 subjects completed the study, and 24 discontinued for various reasons (9 to lack of efficacy; 14 due to nausea and emesis; and 1 for non-related hypotension); and one lost to follow-up.

The study lasted four months and comprised 4 periods: The Screening Period (Days -28 to -1), the Surgical Period (Day 0), the Treatment Period (48 hours; Days 1 to 3) and the Follow-up Period (Days 5 to 9).

The measurements of pain intensity and pain relied were conducted at Time 0 (i.e., at 5, 15, 30, and 45 minutes, and 1, 1.5, 2, 3, 4, 5, 6, 7, 8, 12, 16, 20, 24, 32, 40, and 48 hours).

As agreed with the U.S. Food and Drug Administration ("FDA"), the primary efficacy endpoint in this study was the Summed Pain Intensity Difference relative to baseline over a period of 48 hours (SPID-48). The patient assessment of pain intensity utilized a numeric pain scale (11-point scale with 0 = no pain to 10 = worst possible pain).

The secondary variables were as follows:
SPID over 0 to 4 hours (SPID-4), over 0 to 8 hours (SPID-8), and over 0 to 24 hours (SPID-24) after Time 0;
Time to onset of analgesia (measured as time to perceptible pain relief confirmed by meaningful pain relief using the 2-stopwatch method); and
Pain intensity difference (PID) at each scheduled time point after Time 0.

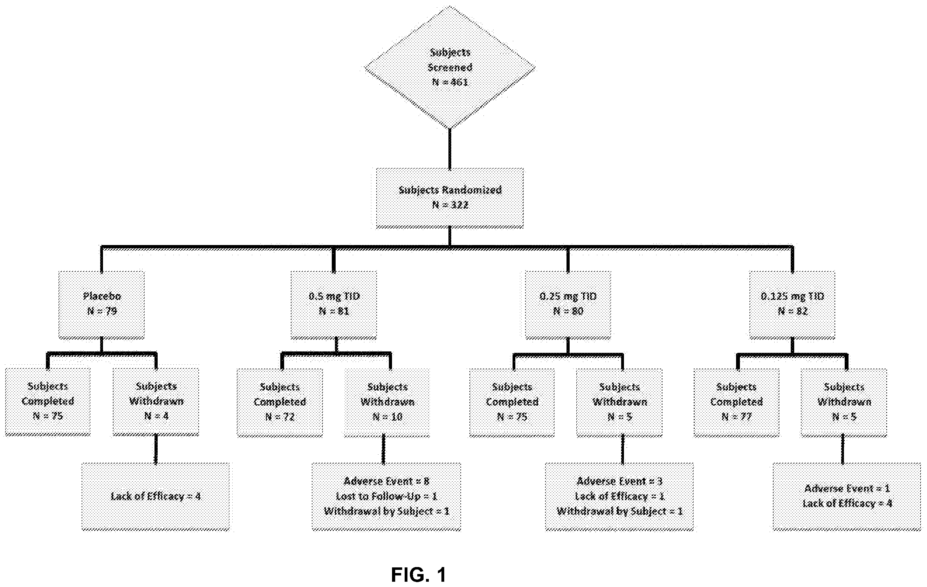

The disposition of subjects is depicted in the flow chart in Figure 1.

### Results

The primary efficacy endpoint was statistically significant at all doses studied. The Buprenorphine Sublingual Spray 0.5 mg tid demonstrated the largest reduction in SPID-48 and was statistically significant to placebo (p<0.0001). The 0.25 mg tid and 0.125 mg tid doses also demonstrated statistically significant reductions in SPID-48 (p = 0.0108 and p = 0.0120, respectively). All treatments were generally well tolerated.

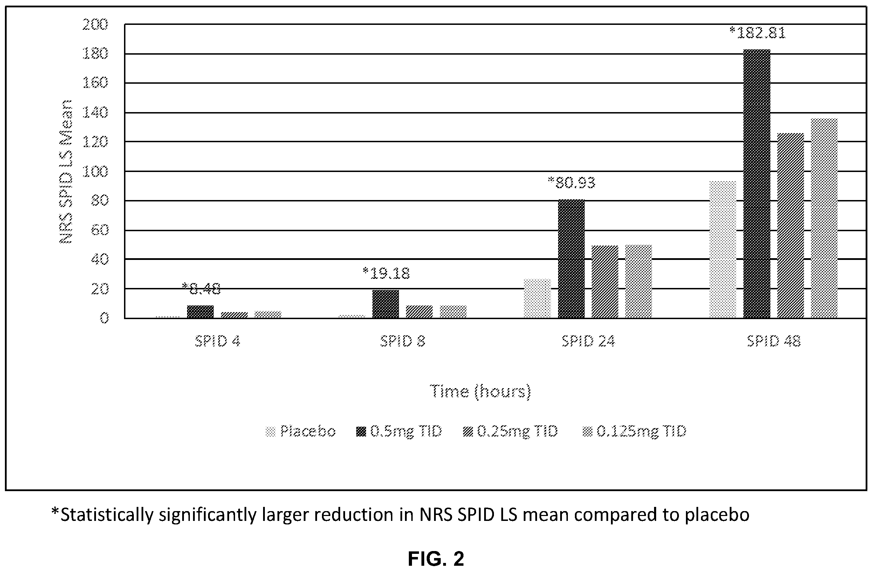

Figure 2 depicts a chart of Numeric Rating Scale (NRS) Summed Pain Intensity Difference (SPID) at 4, 8, 24 and 48 hours.

Table 45 below describes NRS SPID over 0 to 48 hours (NRS SPID-48) for intention-to-treat (ITT) population.

**Table 45**

| Summary of SPID-48 (ITT Population) | | | | |
|---|---|---|---|---|
| Statistic | | Buprenorphine Sublingual Spray | | |
| | Placebo (N=79) | 0.5 mg TID (N=81) | 0.25 mg TID (N=80) | 0.125mg TID (N=82) |
| n | 75 | 72 | 75 | 77 |
| mean (SD) | 93.40 (85.063) | 182.81 (107.349) | 125.75 (102.247) | 135.84 (114.040) |
| CV | 91.07 | 58.72 | 81.31 | 83.95 |
| median | 84.0 | 181.0 | 98.0 | 130.3 |
| min, max | -77.7, 377.8 | -17.8, 414.6 | -55.5, 399.0 | -90.5, 399.4 |
| Least square mean(SE)^{a} | 89.40 (10.109) | 171.33 (10.316) | 125.58 (10.101) | 124.85 (9.944) |
| 95% Cl | 69.50, 109.29 | 151.02, 191.63 | 105.70, 145.46 | 105.28, 144.43 |
| Comparison | Least square mean difference (SE)^{a} | | 95% Cl | P-value^{a} |
| 0.5 mg vs. placebo | 81.93 (14.283) | | 53.82, 110.04 | <0.0001 |
| 0.25 mg vs. placebo | 36.18 (14.099) | | 8.43, 63.93 | 0.0108 |
| 0.125 mg vs. placebo | 35.46 (14.020) | | 7.86, 63.05 | 0.0120 |

| | | | | |
|---|---|---|---|---|
| Note: SPID-48 = Summary of Pain Intensity Differences over 48 hours, CV = coefficient of variation, TID = three times daily. a: Least square means, standard errors(SE), confidence interval(Cl) and p-values are from an ANCOVA model with factors for treatment, site and baseline pain intensity. | | | | |

Table 46 below describes NRS SPID over 0 to 24 hours (NRS SPID-24) for ITT population.

**Table 46**

| Summary of SPID-24 (ITT Population) | | | | |
|---|---|---|---|---|
| Statistic | | Buprenorphine Sublingual Spray | | |
| | Placebo (N=79) | 0.5 mg TID (N=81) | 0.25 mg TID (N=80) | 0.125 mg TID (N=82) |
| n | 75 | 73 | 76 | 77 |
| mean (SD) | 26.61 (42.855) | 80.93 (53.234) | 49.21 (48.223) | 49.90 (56.899) |
| CV | 161.02 | 65.78 | 97.98 | 114.02 |
| median | 21.0 | 83.4 | 43.2 | 48.3 |
| min, max | -46.3, 161.8 | -30.8, 196.9 | -40.9, 177.5 | -62.8,175.9 |
| Least square mean(SE)^{a} | 24.16 (5.001) | 75.67 (5.066) | 48.85 (4.962) | 44.17 (4.920) |
| 95% Cl | 14.31, 34.00 | 65.70, 85.64 | 39.08, 58.62 | 34.49, 53.86 |
| Comparison | Least square mean difference (SE)^{a} | | 95% Cl | P-value^{a} |
| 0.5 mg vs. placebo | 51.51 (7.041) | | 37.66, 65.37 | <0.0001 |
| 0.25 mg vs. placebo | 24.69 (6.952) | | 11.01, 38.38 | 0.0004 |
| 0.125 mg vs. placebo | 20.02 (6.937) | | 6.37, 33.67 | 0.0042 |

| | | | | |
|---|---|---|---|---|
| Note: SPID-24 = Summary of Pain Intensity Differences over 24 hours, CV = coefficient of variation, TID = three times daily. a: Least square means, standard errors(SE), confidence interval(Cl) and p-values are from an ANCOVA model with factors for treatment, site and baseline pain intensity. | | | | |

Table 47 below describes NRS SPID over 0 to 8 hours (NRS SPID-8) for ITT population.

**Table 47**

| Summary of SPID-8 (ITT Population) | | | | |
|---|---|---|---|---|
| Statistic | | Buprenorphine Sublingual Spray | | |
| | Placebo (N=79) | 0.5 mg TID (N=81) | 0.25 mg TID (N=80) | 0.125 mg TID (N=82) |
| n | 77 | 78 | 78 | 78 |
| mean (SD) | 2.14 (13.589) | 19.18 (19.606) | 8.63 (17.661) | 8.71 (18.707) |
| CV | 633.82 | 102.20 | 204.61 | 214.72 |
| median | 0.8 | 19.2 | 7.5 | 6.1 |
| min, max | -25.1, 36.3 | -26.7,65.3 | -23.3, 63.1 | -27.8, 57.2 |
| Least square mean(SE)^{a} | 1.32 (1.851) | 17.57 (1.835) | 8.26 (1.843) | 7.08 (1.837) |
| 95% Cl | -2.32,4.97 | 13.96, 21.18 | 4.63, 11.89 | 3.47, 10.70 |
| Comparison | Least square mean difference (SE)^{a} | | 95% Cl | P-value^{a} |
| 0.5 mg vs. placebo | 16.24 (2.582) | | 11.16, 21.32 | <0.0001 |
| 0.25 mg vs. placebo | 6.93 (2.579) | | 1.86, 12.01 | 0.0076 |
| 0.125 mg vs. placebo | 5.76 (2.582) | | 0.68, 10.84 | 0.0265 |

| | | | | |
|---|---|---|---|---|
| Note: SPID-8 = Summary of Pain Intensity Differences over 8 hours, CV = coefficient of variation, TID = three times daily. a: Least square means, standard errors(SE), confidence interval(Cl) and p-values are from an ANCOVA model with factors for treatment, site and baseline pain intensity. | | | | |

Table 48 below describes NRS SPID over 0 to 4 hours (NRS SPID-4) for ITT population.

**Table 48**

| Summary of SPID-4 (ITT Population) | | | | |
|---|---|---|---|---|
| Statistic | | Buprenorphine Sublingual Spray | | |
| | Placebo (N=79) | 0.5 mg TID (N=81) | 0.25 mg TID (N=80) | 0.125 mg TID (N=82) |
| n | 78 | | 80 | 30 |
| mean (SD) | 1.29 (8.466) | 8.48 (10.089) | 4.15 (9.230) | 4.59 (10.637) |
| CV | 656.18 | 119.05 | 222.41 | 231.79 |
| median | 0.0 | 8.2 | 4.0 | 2.9 |
| min, max | -20.3, 25.3 | -19.1, 302 | -17.2, 27.1 | -22.2, 28.5 |
| Least square mean(SE)^{a} | 0.67 (1.036) | 7.70 (1.013) | 3.67 (1.023) | 3.74 (1.020) |
| 95% Cl | -1.37,2.70 | 5.71, 9.69 | 1.66, 5.68 | 1.73, 5.75 |
| Comparison | Least square mean difference (SE)^{a} | | 95% CI | P-value^{a} |
| 0.5 mg vs. placebo | 7.03 (1.436) | | 4.21, 9.86 | <0.0001 |
| 0.25 mg vs. placebo | 3.00 (1.439) | | 0.17, 5.84 | 0.0377 |
| 0.125 mg vs. placebo | 3.07 (1.441) | | 0.24, 5.91 | 0.0337 |

| | | | | |
|---|---|---|---|---|
| Note: SPID-4 = Summary of Pain Intensity Differences over 4 hours, CV = coefficient of variation, TID = three times daily. a: Least square means, standard errors(SE), confidence interval(Cl) and p-values are from an ANCOVA model with factors for treatment, site and baseline pain intensity. | | | | |

Table 49 shows time of onset analgesia for investigator initiated trials (IIT) population.

**Table 49**

| Time to Onset of Analgesia (ITT Population) | | Buprenorphine Sublingual Spray | | | |
|---|---|---|---|---|---|
| | | Placebo (N=79) | 0.5 mg TID (N=81) | 0.25 mg TID (N=80) | 0.125 mg TID (N=82) |
| Number (%) of subjects with onset of analgesia | | 27 (34.2) | 53 (65.4) | 37 (46.3) | 36 (43.9) |
| Number (%) of subjects censored | | 52 (65.8) | 28 (346) | 43 (53.8) | 46 (56.1) |
| Time (minutes) from first dose to onset of analgesia^{a} | | | | | |
| | 25^{th} percentile (95% Cl) | 5.0 (4.0, 83.0) | 6.0 (5.0, 15.0) | 13.0 (5.0, 29.0) | 15.0 (6.0, 27.0) |
| | Median (95% Cl) | NE | 43.0 (21.0, 64.0) | NE (43.0, NE) | NE (41.0, NE) |
| | 75^{th} percentile (95% Cl) | NE | NE (101.0, NE) | NE | NE |
| | Mean (SE) | 58.4 (4.11) | 146.4 (21.35) | 58.7 (4.46) | 58.3 (4.30) |
| Comparison | | P-value^{b} | | | |
| 0.5 mg vs. placebo | | 0.0010 | | | |
| 0.25 mg vs. placebo | | 0.3018 | | | |
| 0.125 mg vs. placebo | | 0.3701 | | | |

| | | | | | |
|---|---|---|---|---|---|
| a: Percentile estimates and confidence intervals (Cl) are from a Kaplan-Meier analysis. b: P-value from a log-rank test of each treatment arm vs. placebo Note: TID = three times daily, NE = not estimable. Denominator for percentages is the number of subjects per treatment group in the ITT population. Time to onset of analgesia is the time when the first stopwatch is stopped given that the second stopwatch is stopped. If the second stopwatch is not stopped, time will be censored at the time of the second dose of study drug or the use of rescue medication, whichever comes first. If both stopwatches are not stopped, time will be censored at the time of the second dose of study drug or the use of rescue medication whichever comes first. | | | | | |

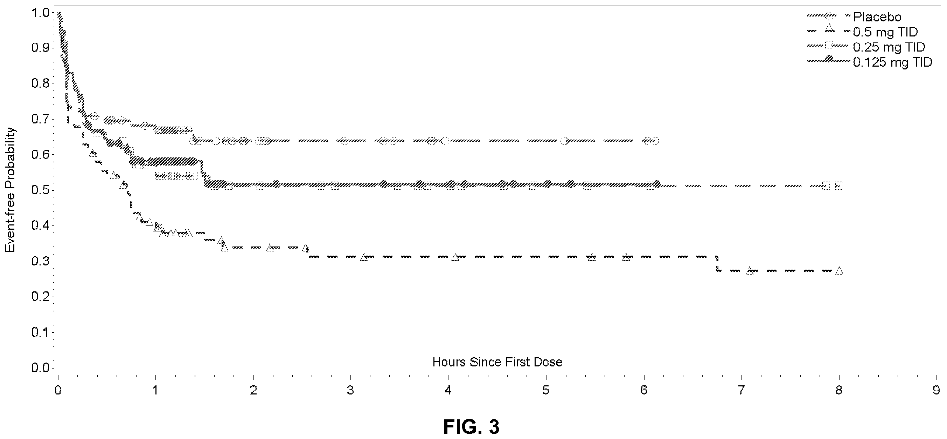

Figure 3 depicts a chart of time of onset of analgesia for placebo, 0.5 mg tid, 0.25 mg tid and 0.125 tid doses.

Table 50 is a representation of mean pain intensity differences by timepoint.

**Table 50**

| **Timepoint** | **Statistic** | **Placebo** | **0.5 mg TID** | **0.2b mg TID** | **0.125 mg TID** |
|---|---|---|---|---|---|
| | | (N=79) | (N=81) | (N=80) | (N=82) |
| 5 minutes | n | 79 | 81 | 80 | 82 |
| | mean (SD) | 0.3 (1.06) | 0.5 (1.15) | 0.3 (1.01) | 0.3 (0.75) |
| 15 minutes | n | 79 | 81 | 80 | 82 |
| | mean (SD) | 0.6 (1.76) | 0.4 (1.39) | 0.6 (1.56) | 0.6 (1.55) |
| 30 minutes | n | 79 | 81 | 80 | 82 |
| | mean (SD) | 0.7 (2.15) | 0.6 (1.65) | 0.7 (2.12) | 0.7 (2.18) |
| 45 minutes | n | 79 | 81 | 80 | 81 |
| | mean (SD) | 0.5 (2.38) | 1.1 (2.08) | 0.9 (2.13) | 1.0 (2.41) |
| 1 hour | n | 79 | 81 | 80 | 81 |
| | mean (SD) | 0.6 (2.58) | 1.5 (2.40) | 1.0 (2.37) | 1.1 (2.62) |
| 1.5 hours | n | 78 | 81 | 80 | 80 |
| | mean (SD) | 0.6 (2.77) | 2.1 (2.72) | 1.2 (2.58) | 1.2 (2.91) |
| 2 hours | n | 78 | 81 | 79 | 80 |
| | mean (SD) | 0.5 (2.77) | 2.4 (3.07) | 1.2 (2.62) | 1.3 (3.05) |
| 3 hours | n | 78 | 81 | 80 | 80 |
| | mean (SD) | 0.2 (2.35) | 2.7 (3.09) | 1.3 (2.95) | 1.3 (3.22) |
| 4 hours | n | 78 | 81 | 80 | 80 |
| | mean (SD) | -0.1 (2.13) | 2.6 (3.26) | 0.9 (3.14) | 1.1 (3.21) |
| 5 hours | n | 77 | 80 | 79 | 80 |
| | mean (SD) | -0.4 (2.08) | 2.6 (3.06) | 0.8 (3.14) | 1.2 (3.32) |
| 6 hours | n | 78 | 78 | 79 | 79 |
| | mean (SD) | 0.2 (2.16) | 3.1 (3.16) | 1.1 (3.16) | 1.1 (3.19) |
| 7 hours | n | 77 | 79 | 79 | 78 |
| | mean (SD) | 0.4 (2.11) | 3.0 (3.06) | 1.3 (2.88) | 0.9 (2.93) |
| 8 hours | n | 77 | 78 | 78 | 78 |
| | mean (SD) | 0.5 (2.10) | 2.5 (3.06) | 1.2 (2.78) | 0.7 (2.73) |
| 12 hours | n | 75 | 78 | 77 | 78 |
| | mean (SD) | 1.0 (2.50) | 3.7 (2.78) | 2.2 (2.88) | 2.0 (3.40) |
| 16 hours | n | 75 | 76 | 76 | 77 |
| | mean (SD) | 0.9 (2.11) | 3.4 (2.65) | 1.9 (2.63) | 1.9 (3.16) |
| 20 hours | n | 75 | 75 | 76 | 77 |
| | mean (SD) | 2.1 (2.90) | 4.3 (2.70) | 3.2 (2.69) | 3.1 (3.07) |
| 24 hours | n | 75 | 73 | 76 | 77 |
| | mean (SD) | 2.2 (2.59) | 4.0 (2.64) | 3.0 (2.72) | 3.2 (2.98) |
| 32 hours | n | 75 | 72 | 75 | 77 |
| | mean (SD) | 2.4 (2.48) | 3.9 (2.89) | 2.9 (2.80) | 3.4 (2.90) |
| 40 hours | n | 75 | 71 | 75 | 77 |
| | mean (SD) | 2.5 (2.21) | 3.9 (2.81) | 3.2 (2.58) | 3.3 (2.80) |
| 48 hours | n | 75 | 72 | 75 | 77 |
| | mean (SD) | 3.5 (2.60) | 4.9 (2.33 | 3.5 (3.00) | 4.1 (2.89) |

The conclusions are as follows:

### Primary efficacy

The largest pain reduction (NRS SPID-48) was observed for the 0.5 mg TID BSS group.

Statistically significantly larger reductions in NRS SPID-48 compared to placebo for the 0.5 mg TID BSS p-value: <0.0001. The largest reduction in NRS SPID-48 compared to placebo was observed for the 0.5 mg TID BSS treatment group.

### Secondary efficacy

Largest pain reductions (NRS SPID-4, NRS SPID-8, and NRS SPID-24) were observed for 0.5 mg TID BSS group (p-value: <0.0001). Secondary time points at 4, 8 and 24 hours SPID were all statistically significantly different.

### Example 9: Pharmacokinetic Data for Formulation 20

### Objective

The primary objective of this study was to compare the bioavailability of a test formulation of Buprenorphine-Naloxone Sublingual (SL) spray, 6.5 mg/1.63 mg (1 spray) to that of a single dose of Suboxone^{®} (buprenorphine and naloxone) sublingual film, 12 mg/3 mg, under fasted conditions. The secondary objective was to evaluate the safety and tolerability of Buprenorphine-Naloxone SL spray.

### Study Design

This was a single-dose, open-label, randomized, two-period, two-treatment crossover study. Fifty-six healthy subjects were enrolled. Subjects who successfully completed the screening process checked into the research center the evening before first dose. Subjects who continued to meet inclusion/exclusion criteria the morning of dose were assigned a subject number, based on the order in which they successfully completed the screening process and procedures as outlined in the study protocol. Subjects were randomly assigned to a treatment sequence and received two separate single-dose administrations of study medication, one treatment per period, according to the randomization schedule. Dosing days were separated by a washout period of at least 14 days.

Subjects received each of the treatments listed below during the two treatment periods:
Treatment A: Test Product
Buprenorphine Naloxone SL spray, 6.5 mg/1.63 mg
Dose = 1 sublingual spray (total dose 6.5 mg/1.63 mg)
Treatment B: Reference Product
   Suboxone^{®} (buprenorphine and naloxone) sublingual film, 12 mg/3 mg
Dose = 1 × 12 mg/3 mg sublingual film

### Clinical Procedures Summary

During each study period, 6 mL blood samples were obtained for buprenorphine, norbuprenorphine, and unconjugated naloxone analysis before and after each dose at selected times through 144 hours after dose administration. A total of 34 pharmacokinetic (PK) blood samples were collected from each subject for buprenorphine, norbuprenorphine, and unconjugated naloxone, 17 samples in each study period. In addition, 6 mL blood samples were obtained for total naloxone analysis before and after each dose at selected times through 72 hours after dose administration. A total of 28 PK blood samples were collected from each subject for naloxone analysis, 14 samples in each study period.

### Procedures for Collecting Samples for Pharmacokinetic Analysis

Blood samples (1 × 6 mL) for buprenorphine, norbuprenorphine, and unconjugated naloxone analysis were collected at 0 (predose), and at 5 minutes, 10 minutes, 15 minutes, 30 minutes, and 1, 2, 4, 8, 12, 24, 36, 48, 72, 96, 120, and 144 hours.

Blood samples (1 × 6 mL) for total naloxone analysis were collected at 0 (predose), and at 5 minutes, 10 minutes, 15 minutes, 30 minutes, and 1, 2, 4, 8, 12, 24, 36, 48, and 72 hours.

### Bioanalytical Summary

Plasma samples were analyzed for buprenorphine, norbuprenorphine, unconjugated naloxone, and total naloxone by Worldwide Clinical Trials (WCT) using validated LC-MS-MS procedures. The methods were validated for ranges of 20.0 to 10,000 pg/mL for buprenorphine and norbuprenorphine and 2.00 to 1000 pg/mL for unconjugated naloxone, based on the analysis of 1.00 mL of human EDTA plasma, and 0.0500 to 50.0 ng/mL for total naloxone, based on the analysis of 0.200 mL of human EDTA plasma. Data were stored in Watson Laboratory Information Management System (LIMS; Version 7.2.0.03, Thermo Fisher Scientific).

### Pharmacokinetic Analysis

Concentration-time data were analyzed using noncompartmental methods in Phoenix^{™} WinNonlin^{®} (Version 6.3, Pharsight Corporation). Concentration-time data that were below the limit of quantification (BLQ) were treated as zero in the data summarization and descriptive statistics. In the pharmacokinetic analysis, BLQ concentrations were treated as zero from time- zero up to the time at which the first quantifiable concentration was observed; embedded and/or terminal BLQ concentrations were treated as "missing". Actual sample times were used in the pharmacokinetic analysis. The linear trapezoidal method was used to calculation the area under the curve (AUC).

The following pharmacokinetic parameters were calculated: peak concentration in plasma (Cₘₐₓ), time to peak concentration (Tₘₐₓ), elimination rate constant (λ_{z}), terminal half-life (T_{1/2}), area under the concentration-time curve from time-zero to the time of the last quantifiable concentration (AUC)ₗₐₛₜ), area under the plasma concentration time curve from time-zero extrapolated to infinity (AUC_{inf}), the percent of AUC_{inf} based on extrapolation (AUCₑₓₜᵣₐₚ), last quantifiable plasma concentration (Cₗₐₛₜ), and time of the last quantifiable plasma concentration (Tₗₐₛₜ). In addition, partial AUCs AUC₀₋₇₂, AUC₀₋₉₆, AUC₀-₁₂₀, and AUC₀₋₁₄₄ were estimated for buprenorphine and unconjugated naloxone to provide information regarding systemic exposure at different times during the extended pharmacokinetic sampling interval.

Analysis of variance (ANOVA) and the Schuirmann's two one-sided t-test procedure at the 5% significance level were applied to the log-transformed pharmacokinetic exposure parameters, Cₘₐₓ, AUCₗₐₛₜ, and AUC_{inf} for buprenorphine, norbuprenorphine, unconjugated naloxone, and total naloxone. The ratio of the geometric means (Insys Sublingual Spray-Test/Suboxone Sublingual Film-Reference) was reported along with the 90% confidence interval about the ratio. For informational purposes, AUC₀₋₇₂, AUC₀₋₉₆, AUC₀₋₁₂₀, and AUC₀₋₁₄₄ for buprenorphine and unconjugated naloxone were compared across treatments using an analogous statistical method.

### Results and Discussion

Data from 50 subjects who completed at least one study period were included in the pharmacokinetic and statistical analyses. Mean concentration-time data are shown in Tables 51 through 54. Results of the pharmacokinetic and statistical analyses are shown below in Tables 55 through 64.

### Buprenorphine

Overall, the pharmacokinetic profile of buprenorphine after the administration of Buprenorphine Naloxone SL spray, 6.5 mg/1.63 mg was similar to that after the administration of Suboxone Sublingual Film 12 mg/3 mg. From the mean buprenorphine concentration-time profiles, the concentrations achieved after the Sublingual Spray were comparable to those after Suboxone, even though a much lower Sublingual Spray dose was administered (6.5 mg in Sublingual Spray vs. 12 mg in Suboxone). At early time points and through approximately 24 hours, the mean buprenorphine concentration-time profiles were practically superimposable for the two treatments; at latter time points, minor differences were noted, with the mean buprenorphine concentrations after the Sublingual Spray being slightly lower than those after Suboxone. These trends were reflected in the derived pharmacokinetic parameters. No appreciable differences were noted in the mean buprenorphine Cₘₐₓ across treatments (5670 ± 1590 pg/mL after Sublingual Spray, 6210 ± 3110 pg/mL after Suboxone). No appreciable differences were noted in the mean ± SD buprenorphine AUC₀₋₇₂, AUC₀₋₉₆, AUC₀₋₁₄₄, AUCₗₐₛₜ, and AUC_{inf}. For example, mean AUCₗₐₛₜ was 46660 ± 12980 h*pg/mL after Sublingual Spray and 56100 ± 21460 h^{∗}pg/mL after Suboxone. Due to the extended pharmacokinetic sampling interval used in this study, AUC to the last quantifiable sample (AUCₗₐₛₜ) provided a reasonable estimate of the overall systemic exposure (AUC_{inf}, extrapolated to infinity). Mean AUC_{inf} values were 48790 ± 13810 h*pg/mL after Sublingual Spray and 59240 ± 22500 h*pg/mL after Suboxone. On average, only 4.27 to 5.28% of AUC_{inf} was based on extrapolation.

It should be noted that some degree of pharmacokinetic variability was observed, in particular for Suboxone relative to that for the Sublingual Spray; the intersubject variability (CV%) for Cₘₐₓ and AUCs ranged from 27.81 to 28.31% for the Sublingual Spray and 37.98 to 50.02% for Suboxone. It was also noted that a differential location shift existed between the mean and median AUC values for Suboxone; the mean AUClast and AUCinf values for Suboxone were higher than the median, suggesting that the data were skewed toward the upper range. The differential distribution of the AUC_{inf} values between the two treatments may have contributed to the ANOVA results for this metric (discussed below).

From the statistical analysis log-transformed pharmacokinetic parameters using an ANOVA model, the geometric mean ratios (90% confidence interval) for buprenorphine Cₘₐₓ, AUCₗₐₛₜ, and AUC_{inf} were 96.01% (88.29, 104.42%), 86.11% (80.44, 92.18%), and 85.19% (79.64, 91.12%), respectively. The ANOVA results for buprenorphine AUC₀₋₇₂, AUC₀₋₉₆, AUC₀₋₁₂₀, and AUC₀₋₁₄₄ were 88.40% (82.59, 94.62%), 87.37% (81.68, 93.46%), 86.75% (81.12, 92.77%), and 86.31% (80.72, 92.29%), respectively. Hence, based on actual data over the 144-hour sampling interval (and over truncated intervals through 72, 96, 120 and 144 hours), bioequivalence criteria were met for buprenorphine in comparisons of the Sublingual Spray to Suboxone. The lower 90% confidence interval for the extrapolated AUC (AUC_{inf}) was 79.64%, 0.36% below the standard bioequivalence limit (80.00%) using the two one-sided tests procedure.

### Norbuprenorphine

Exposure to norbuprenorphine differed across treatments. Based on mean estimates of Cₘₐₓ and AUCs, exposure to norbuprenorphine was 2- to 2.6-fold lower after the Sublingual Spray relative to Suboxone, possibly due to increased direct absorption into systemic circulation and lower presystemic, first-pass metabolism for the Sublingual Spray.

### Unconjugated Naloxone

Overall, the pharmacokinetic profile of unconjugated naloxone after the administration of Buprenorphine Naloxone SL spray, 6.5 mg/1.63 mg was similar to that after the administration of Suboxone Sublingual Film 12 mg/3 mg. Based on mean estimates of Cₘₐₓ and AUCs, exposure to unconjugated naloxone was comparable across treatments. Mean Cₘₐₓ was 379 ± 211 pg/mL after Sublingual Spray and 356 ± 149 pg/mL after Suboxone; mean AUCₗₐₛₜ was 887.6 ± 445.4 h*pg/mL after Sublingual Spray and 942.0 ± 430.1 h*pg/mL after Suboxone. AUC_{inf} were similar to AUCₗₐₛₜ values; due to the relatively short T_{1/2} of unconjugated naloxone (approximately 3 to 4 hours), only 2.18 to 2.41% of AUC_{inf} was based on extrapolation.

From the statistical analysis log-transformed pharmacokinetic parameters using an ANOVA model, the geometric mean ratios (90% confidence interval) for unconjugated naloxone Cₘₐₓ, AUCₗₐₛₜ, and AUC_{inf} were 103.72% (93.78, 114.71%), 94.95% (86.93, 103.72%), and 94.69% (86.79, 103.31%), respectively. The ANOVA results for unconjugated naloxone AUC₀₋₇₂, AUC₀₋₉₆, AUC₀₋₁₂₀, and AUC₀₋₁₄₄ were comparable to those for AUCₗₐₛₜ and AUC_{inf}. Hence, bioequivalence criteria were met for all pharmacokinetic metrics considered in the analysis.

### Total Naloxone

Exposure to total naloxone differed across treatments. Based on mean estimates of Cₘₐₓ and AUCs, exposure to total naloxone was approximately 2-fold lower after the Sublingual Spray relative to Suboxone, possibly due to increased direct absorption into systemic circulation and lower presystemic, first-pass metabolism/glucuronidation for the Sublingual Spray.

### Conclusions

Overall, the pharmacokinetic profile of buprenorphine after the administration of Buprenorphine Naloxone SL spray, 6.5 mg/1.63 mg was similar to that after the administration of Suboxone Sublingual Film 12 mg/3 mg. No significant differences in Cₘₐₓ and AUCs over the 144-hour pharmacokinetic sampling period were observed and bioequivalence criteria (90% confidence intervals within 80.00-125.00%) were met for the AUC at 72 hours (82.6% - 94.6%), 96 hours (81.7% - 93.5%), 120 hours (81.1% - 92.8%), and 144 hours (80.7% - 92.3%) postdose. The lower 90% confidence interval for the extrapolated AUC (AUC_{inf}) was 79.64%, 0.36% below the bioequivalence limit of 80.00%. Therefore, based on data acquired over an extended sampling period (144 hours or 6 days), Buprenorphine Naloxone SL spray, 6.5 mg/1.63 mg is considered essentially bioequivalent to Sublingual Film 12 mg/3 mg.

The pharmacokinetic profile of unconjugated naloxone after the administration of Buprenorphine Naloxone SL spray, 6.5 mg/1.63 mg was similar to that after the administration of Suboxone Sublingual Film 12 mg/3 mg. No significant differences in Cₘₐₓ and AUCs were observed and bioequivalence criteria (90% confidence intervals within 80.00-125.00%) were met for all pharmacokinetic metrics considered in the analysis.

**Table 51: Buprenorphine Concentration-Time Data after Administration of the Test Product (Treatment A) and the Reference Product (Treatment B).**

| **Time (h)** | **Treatment A:** | | | | **Treatment B:** | | | |
|---|---|---|---|---|---|---|---|---|
| | **Test Product** | | | | **Reference Product (Suboxone)** | | | |
| | **n** | **Mean (pg/mL)** | **SD (pg/mL)** | **CV (%)** | **n** | **Mean (pg/mL)** | **SD (pg/mL)** | **CV (%)** |
| **0.00** | 50 | 0.00 | 0.00 | NC | 49 | 0.00 | 0.00 | NC |
| **0.08** | 49 | 36.9 | 66.2 | 179.77 | 49 | 1.30 | 9.13 | 700.00 |
| **0.17** | 50 | 491 | 463 | 94.30 | 49 | 50.2 | 73.9 | 147.10 |
| **0.25** | 50 | 1220 | 902 | 74.02 | 49 | 254 | 255 | 100.57 |
| **0.50** | 50 | 3270 | 1570 | 48.13 | 49 | 2300 | 1690 | 73.45 |
| **1.00** | 50 | 4990 | 1690 | 33.93 | 49 | 5130 | 3060 | 59.61 |
| **2.00** | 50 | 5420 | 1530 | 28.14 | 49 | 5440 | 2300 | 42.27 |
| **4.00** | 50 | 3580 | 1270 | 35.49 | 49 | 3660 | 2080 | 56.97 |
| **8.00** | 50 | 1150 | 407 | 35.36 | 49 | 1360 | 983 | 72.21 |
| **12.00** | 50 | 576 | 181 | 31.50 | 49 | 798 | 423 | 53.06 |
| **24.00** | 50 | 293 | 90.6 | 30.94 | 49 | 448 | 166 | 37.07 |
| **36.00** | 50 | 212 | 73.7 | 34.76 | 49 | 329 | 121 | 36.70 |
| **48.00** | 50 | 144 | 53.2 | 36.84 | 49 | 218 | 86.4 | 39.56 |
| **72.00** | 50 | 88.8 | 37.2 | 41.95 | 49 | 133 | 54.2 | 40.70 |
| **96.00** | 50 | 59.9 | 28.1 | 46.83 | 49 | 87.6 | 40.6 | 46.40 |
| **120.00** | 50 | 37.6 | 25.0 | 66.57 | 49 | 59.2 | 31.1 | 52.45 |
| **144.00** | 50 | 25.5 | 21.7 | 85.24 | 48 | 42.1 | 30.9 | 73.38 |

| | | | | | | | | |
|---|---|---|---|---|---|---|---|---|
| Note: Plasma samples analyzed using a bioanalytical method with a validated range 20.0 to 10,000 pg/mL; concentrations reported in pg/mL to 3 significant figures; concentrations below limit of quantification set to zero (0.00 pg/mL) in the data summarization NC = Not calculated | | | | | | | | |

**Table 52: Norbuprenorphine Concentration-Time Data after Administration of the Test Product (Treatment A) and the Reference Product (Treatment B).**

| **Time (h)** | **Treatment A:** | | | | **Treatment B:** | | | |
|---|---|---|---|---|---|---|---|---|
| | **Test Product** | | | | **Reference Product (Suboxone)** | | | |
| | **n** | **Mean (pg/mL)** | **SD (pg/mL)** | **CV (%)** | **n** | **Mean (pg/mL)** | **SD (pg/mL)** | **CV (%)** |
| **0.00** | 50 | 0.418 | 2.96 | 707.11 | 49 | 0.567 | 3.97 | 700.00 |
| **0.08** | 49 | 1.04 | 5.09 | 489.80 | 49 | 0.651 | 4.56 | 700.00 |
| **0.17** | 50 | 30.4 | 60.1 | 197.69 | 49 | 3.59 | 12.7 | 353.54 |
| **0.25** | 50 | 137 | 203 | 147.89 | 49 | 41.1 | 89.5 | 217.66 |
| **0.50** | 50 | 456 | 421 | 92.37 | 49 | 800 | 1000 | 125.44 |
| **1.00** | 50 | 684 | 478 | 69.85 | 49 | 1990 | 1650 | 82.59 |
| **2.00** | 50 | 740 | 415 | 56.01 | 49 | 1800 | 1080 | 60.10 |
| **4.00** | 50 | 614 | 304 | 49.60 | 49 | 1260 | 674 | 53.60 |
| **8.00** | 50 | 522 | 235 | 45.03 | 49 | 1020 | 535 | 52.18 |
| **12.00** | 50 | 470 | 217 | 46.21 | 49 | 945 | 469 | 49.62 |
| **24.00** | 50 | 466 | 199 | 42.64 | 49 | 984 | 482 | 49.04 |
| **36.00** | 50 | 390 | 150 | 38.38 | 49 | 828 | 384 | 46.44 |
| **48.00** | 50 | 304 | 132 | 43.32 | 49 | 633 | 302 | 47.76 |
| **72.00** | 50 | 212 | 107 | 50.44 | 49 | 435 | 234 | 53.80 |
| **96.00** | 50 | 151 | 87.5 | 57.93 | 49 | 302 | 172 | 56.88 |
| **120.00** | 50 | 108 | 74.4 | 68.68 | 49 | 213 | 141 | 65.91 |
| **144.00** | 50 | 86.3 | 67.1 | 77.79 | 48 | 171 | 132 | 77.70 |

| | | | | | | | | |
|---|---|---|---|---|---|---|---|---|
| Note: Plasma samples analyzed using a bioanalytical method with a validated range 20.0 to 10,000 pg/mL; concentrations reported in pg/mL to 3 significant figures; concentrations below limit of quantification set to zero (0.00 pg/mL) in the data summarization | | | | | | | | |

**Table 53: Unconjugated Naloxone Concentration-Time Data after Administration of the Test Product (Treatment A) and the Reference Product (Treatment B).**

| **Time (h)** | **Treatment A:** | | | | **Treatment B:** | | | |
|---|---|---|---|---|---|---|---|---|
| | **Test Product** | | | | **Reference Product (Suboxone)** | | | |
| | **n** | **Mean (pg/mL)** | **SD (pg/mL)** | **CV (%)** | **n** | **Mean (pg/mL)** | **SD (pg/mL)** | **CV (%)** |
| **0.00** | 50 | 0.00 | 0.00 | NC | 49 | 0.00 | 0.00 | NC |
| **0.08** | 49 | 50.4 | 51.9 | 103.09 | 49 | 5.08 | 17.7 | 349.10 |
| **0.17** | 50 | 205 | 171 | 83.54 | 49 | 47.5 | 75.0 | 157.82 |
| **0.25** | 50 | 292 | 232 | 79.37 | 49 | 105 | 99.0 | 94.56 |
| **0.50** | 50 | 349 | 199 | 57.08 | 49 | 294 | 164 | 55.74 |
| **1.00** | 50 | 293 | 140 | 47.98 | 49 | 304 | 120 | 39.42 |
| **2.00** | 50 | 166 | 84.8 | 50.93 | 49 | 177 | 86.5 | 48.78 |
| **4.00** | 50 | 54.3 | 30.3 | 55.76 | 49 | 66.8 | 64.8 | 96.90 |
| **8.00** | 50 | 9.06 | 4.90 | 54.14 | 49 | 14.3 | 15.7 | 109.92 |
| **12.00** | 50 | 3.67 | 3.56 | 97.16 | 49 | 6.80 | 6.47 | 95.25 |
| **24.00** | 50 | 0.705 | 1.87 | 265.19 | 49 | 2.14 | 3.18 | 148.44 |
| **36.00** | 50 | 0.00 | 0.00 | NC | 49 | 0.219 | 0.749 | 341.25 |
| **48.00** | 50 | 0.00 | 0.00 | NC | 49 | 0.00 | 0.00 | NC |
| **72.00** | 50 | 0.00 | 0.00 | NC | 49 | 0.00 | 0.00 | NC |
| **96.00** | 50 | 0.00 | 0.00 | NC | 49 | 0.00 | 0.00 | NC |
| **120.00** | 49 | 0.00 | 0.00 | NC | 49 | 0.00 | 0.00 | NC |
| **144.00** | 50 | 0.00 | 0.00 | NC | 48 | 0.00 | 0.00 | NC |

| | | | | | | | | |
|---|---|---|---|---|---|---|---|---|
| Note: Plasma samples analyzed using a bioanalylical method with a validated range 2.00 to 1000 pg/mL; concentrations reported in pg/mL to 3 significant figures; concentrations below limit of quantification set to zero (0.00 pg/mL) in the data summarization NC = Not calculated | | | | | | | | |

**Table 54: Total Naloxone Concentration-Time Data after Administration of the Test Product (Treatment A) and the Reference Product (Treatment B).**

| **Time (h)** | **Treatment A:** | | | | **Treatment B:** | | | |
|---|---|---|---|---|---|---|---|---|
| | **Test Product** | | | | **Reference Product (Suboxone)** | | | |
| | **n** | **Mean (ng/mL)** | **SD (ng/mL)** | **CV (%)** | **n** | **Mean (ng/mL)** | **SD (ng/mL)** | **CV (%)** |
| **0.00** | 50 | 0.00 | 0.00 | NC | 49 | 0.00 | 0.00 | NC |
| **0.08** | 49 | 0.150 | 0.275 | 183.48 | 49 | 0.0285 | 0.0991 | 347.54 |
| **0.17** | 50 | 1.44 | 1.81 | 125.87 | 49 | 0.416 | 0.976 | 234.70 |
| **0.25** | 50 | 4.14 | 4.12 | 99.53 | 49 | 2.50 | 4.94 | 197.40 |
| **0.50** | 50 | 8.90 | 6.50 | 73.10 | 49 | 15.7 | 14.1 | 89.82 |
| **1.00** | 50 | 9.19 | 4.45 | 48.44 | 49 | 21.3 | 10.2 | 48.03 |
| **2.00** | 50 | 5.02 | 2.75 | 54.78 | 49 | 9.76 | 4.53 | 46.43 |
| **4.00** | 50 | 1.50 | 0.960 | 64.13 | 49 | 2.67 | 1.33 | 49.92 |
| **8.00** | 50 | 0.846 | 0.554 | 65.50 | 49 | 1.42 | 1.10 | 77.40 |
| **12.00** | 50 | 0.626 | 0.688 | 109.87 | 49 | 1.05 | 0.514 | 48.83 |
| **24.00** | 50 | 0.211 | 0.121 | 57.26 | 49 | 0.428 | 0.281 | 65.62 |
| **36.00** | 50 | 0.0583 | 0.0680 | 116.78 | 49 | 0.126 | 0.0975 | 77.13 |
| **48.00** | 50 | 0.0101 | 0.0281 | 278.20 | 49 | 0.0520 | 0.0733 | 141.05 |
| **72.00** | 50 | 0.00110 | 0.00778 | 707.11 | 49 | 0.00263 | 0.0129 | 490.72 |

| | | | | | | | | |
|---|---|---|---|---|---|---|---|---|
| Note: Plasma samples analyzed using a bioanalytical method with a validated range 0.0500 to 50.0 ng/mL; concentrations reported in ng/mL to 3 significant figures; concentrations below limit of quantification set to zero (0.00 ng/mL) in the data summarization NC = Not calculated | | | | | | | | |

**Table 55: Pharmacokinetic Parameters of Buprenorphine.**

| **Parameter** | **Treatment A:** | | | | **Treatment B:** | | | |
|---|---|---|---|---|---|---|---|---|
| | **Test Product** | | | | **Reference Product (Suboxone)** | | | |
| | **n** | **Mean** | **SD** | **CV%** | **n** | **Mean** | **SD** | **CV%** |
| **Tₘₐₓ (h)** | 50 | 1.63 | 0.50 | 30.77 | 49 | 1.66 | 0.72 | 43.56 |
| **Median (Range)** | | 2.00 | (0.50 - 2.00) | | | 2.00 | (0.50 - 4.00) | |
| **Cₘₐₓ (pg/mL)** | 50 | 5670 | 1590 | 28.08 | 49 | 6210 | 3110 | 50.02 |
| **AUCₗₐₛₜ (h*pg/mL)** | 50 | 46660 | 12980 | 27.81 | 49 | 56100 | 21460 | 38.25 |
| **AUC_{inf} (h*pg/mL)** | 50 | 48790 | 13810 | 28.31 | 49 | 59240 | 22500 | 37.98 |
| **AUC₀₋₇₂ (h*pg/mL)** | 50 | 43040 | 11670 | 27.11 | 49 | 50560 | 19670 | 38.91 |
| **AUC₀₋₉₆ (h*pg/mL)** | 50 | 44830 | 12140 | 27.07 | 49 | 53210 | 20390 | 38.32 |
| **AUC₀₋₁₂₀ (h*pg/mL)** | 50 | 46030 | 12500 | 27.16 | 49 | 54980 | 20910 | 38.04 |
| **AUC₀₋₁₄₄ (h*pg/mL)** | 50 | 46860 | 12790 | 27.30 | 49 | 56230 | 21320 | 37.92 |
| **AUC_{Extrap} (%)** | 50 | 4.27 | 2.13 | 49.83 | 49 | 5.28 | 3.01 | 57.02 |
| **λ_{z} (h⁻¹)** | 50 | 0.0175 | 0.0043 | 24.51 | 49 | 0.0172 | 0.0041 | 23.74 |
| **T_{1/2} (h)** | 50 | 41.84 | 10.15 | 24.26 | 49 | 42.72 | 10.38 | 24.30 |
| **Tₗₐₛₜ (h)** | 50 | 133.44 | 18.24 | 13.67 | 49 | 137.60 | 14.50 | 10.54 |
| **Cₗₐₛₜ (pg/mL)** | 50 | 33.2 | 15.3 | 46.10 | 49 | 47.2 | 24.8 | 52.46 |

**Table 56: Pharmacokinetic Parameters of Norbuprenorphine**

| **Parameter** | **Treatment A:** | | | | **Treatment B:** | | | |
|---|---|---|---|---|---|---|---|---|
| | **Test Product** | | | | **Reference Product (Suboxone)** | | | |
| | **n** | **Mean** | **SD** | **CV%** | **n** | **Mean** | **SD** | **CV%** |
| **Tₘₐₓ (h)** | 50 | 3.49 | 5.54 | 158.73 | 49 | 3.98 | 7.39 | 185.67 |
| **Median (Range)** | | 2.00 | (0.50 - 24.00) | | | 1.00 | (0.50 - 36.00) | |
| **Cₘₐₓ (pg/mL)** | 50 | 854 | 461 | 53.99 | 49 | 2220 | 1540 | 69.12 |
| **AUCₗₐₛₜ (h^{*}pg/mL)** | 50 | 37570 | 14560 | 38.75 | 49 | 77800 | 34820 | 44.76 |
| **AUC_{inf} (h*pg/mL)** | 50 | 46870 | 22370 | 47.72 | 49 | 93460 | 47480 | 50.81 |
| **AUC_{Extrap} (%)** | 50 | 16.39 | 13.43 | 81.94 | 49 | 14.15 | 10.41 | 73.53 |
| **λ_{Z} (h⁻¹)** | 50 | 0.0159 | 0.0076 | 47.52 | 49 | 0.0159 | 0.0060 | 37.45 |
| **T_{1/2} (h)** | 50 | 56.50 | 34.49 | 61.05 | 49 | 50.97 | 23.27 | 45.66 |
| **Tₗₐₛₜ (h)** | 50 | 141.12 | 9.25 | 6.56 | 49 | 143.48 | 3.42 | 2.39 |
| **Cₗₐₛₜ (pg/mL)** | 50 | 89.3 | 63.8 | 71.43 | 49 | 173 | 132 | 76.44 |

**Table 57: Pharmacokinetic Parameters of Unconjugated Naloxone**

| **Parameter** | **Treatment A:** | | | | **Treatment B:** | | | |
|---|---|---|---|---|---|---|---|---|
| | **Test Product** | | | | **Reference Product (Suboxone) n** | | | |
| | **n** | **Mean** | **SD** | **CV%** | | **Mean** | **SD** | **CV%** |
| **Tₘₐₓ (h)** | 50 | 0.56 | 0.28 | 50.10 | 49 | 0.84 | 0.56 | 66.81 |
| **Median (Range)** | | 0.50 | (0.17 - 1.03) | | | 1.00 | (0.25 - 4.00) | |
| **Cmax (pg/mL)** | 50 | 379 | 211 | 55.76 | 49 | 356 | 149 | 41.75 |
| **AUCₗₐₛₜ (h*pg/mL)** | 50 | 887.6 | 445.4 | 50.18 | 49 | 942.0 | 430.1 | 45.66 |
| **AUC_{inf} (h*pg/mL)** | 50 | 904.9 | 445.9 | 49.27 | 48 | 942.0 | 411.0 | 43.63 |
| **AUC₀₋₇₂ (h*pg/mL)** | 50 | 903.4 | 446.3 | 49.40 | 48 | 941.1 | 410.6 | 43.63 |
| **AUC₀₋₉₆ (h*pg/mL)** | 50 | 904.0 | 446.1 | 49.35 | 48 | 941.6 | 410.9 | 43.64 |
| **AUC₀₋₁₂₀ (h*pg/mL)** | 50 | 904.3 | 446.0 | 49.32 | 48 | 941.7 | 411.0 | 43.64 |
| **AUC₀₋₁₄₄ (h*pg/mL)** | 50 | 904.5 | 445.9 | 49.30 | 48 | 941.8 | 411.0 | 43.64 |
| **AUC_{Extrap} (%)** | 50 | 2.18 | 2.79 | 128.30 | 48 | 2.41 | 1.33 | 55.07 |
| **λ_{Z} (h⁻¹)** | 50 | 0.3617 | 0.1584 | 43.79 | 48 | 0.2547 | 0.1450 | 56.93 |
| **T_{1/2} (h)** | 50 | 3.48 | 5.51 | 158.21 | 48 | 4.15 | 3.07 | 74.06 |
| **Tₗₐₛₜ (h)** | 50 | 13.44 | 5.58 | 41.55 | 49 | 18.37 | 8.28 | 45.10 |
| **Cₗₐₛₜ (pg/mL)** | 50 | 4.00 | 1.90 | 47.39 | 49 | 4.51 | 3.29 | 72.93 |

**Table 58: Pharmacokinetic Parameters of Total Naloxone**

| **Parameter** | **Treatment A:** | | | | **Treatment B:** | | | |
|---|---|---|---|---|---|---|---|---|
| | **Test Product** | | | | **Reference Product (Suboxone)** | | | |
| | **n** | **Mean** | **SD** | **CV%** | **n** | **Mean** | **SD** | **CV%** |
| **Tₘₐₓ (h)** | 50 | 1.40 | 2.13 | 152.59 | 49 | 1.12 | 1.14 | 101.89 |
| **Median (Range)** | | 1.00 | (0.25 - 12.00) | | | 1.00 | (0.50 - 8.00) | |
| **Cₘₐₓ (ng/mL)** | 50 | 12.0 | 5.38 | 44.86 | 49 | 24.9 | 11.9 | 47.75 |
| **AUCₗₐₛₜ (h^{*}ng/mL)** | 50 | 34.12 | 10.51 | 30.80 | 49 | 65.80 | 20.43 | 31.04 |
| **AUC_{inf} (h*ng/mL)** | 48 | 36.22 | 10.45 | 28.84 | 49 | 66.99 | 20.39 | 30.44 |
| **AUC_{Extrap} (%)** | 48 | 4.48 | 2.92 | 65.21 | 49 | 2.31 | 2.89 | 124.97 |
| **λ_{z} (h⁻¹)** | 48 | 0.0911 | 0.0377 | 41.37 | 49 | 0.0923 | 0.0277 | 30.03 |
| **T_{1/2} (h)** | 48 | 8.71 | 3.31 | 38.00 | 49 | 8.24 | 2.71 | 32.85 |
| **Tₗₐₛₜ (h)** | 50 | 32.16 | 10.41 | 32.37 | 49 | 41.14 | 10.95 | 26.63 |
| **Cₗₐₛₜ (ng/mL)ANO** | 50 | 0.122 | 0.0587 | 48.20 | 49 | 0.0987 | 0.0575 | 58.20 |

**Table 59: Statistical Analysis of the Log-Transformed Systemic Exposure Parameters of Buprenorphine**

| **Dependent Variable** | **Geometric Mean^{a}** | | **Ratio (%)^{b}** | **90% CI^{c}** | | **Power** | ANOVA |
|---|---|---|---|---|---|---|---|
| | **Test** | **Ref** | **(Test/Ref)** | **Lower** | **Upper** | | **CV%** |
| **In(Cₘₐₓ)** | 5431.5970 | 5657.0376 | 96.01 | 88.29 | 104.42 | 0.9961 | 23.69 |
| **ln(AUCₗₐₛₜ**) | 45456.8371 | 52788.4107 | 86.11 | 80.44 | 92.18 | 0.9998 | 19.14 |
| **In(AUC_{inf})** | 47445.5869 | 55696.4070 | 85.19 | 79.64 | 91.12 | 0.9998 | 18.91 |

| | | | | | | | |
|---|---|---|---|---|---|---|---|
| ^{a} Geometric Mean for the Test Product (Test) and Reference Product (Ref) based on Least Squares Mean of log-transformed parameter values ^{b} Ratio(%) = Geometric Mean (Test)/Geometric Mean (Ref) ^{c} 90% Confidence Interval | | | | | | | |

**Table 60: Statistical Analysis of the Log-Transformed Systemic Exposure Parameters of Norbuprenorphine**

| **Dependent Variable** | **Geometric Mean^{a}** | | **Ratio (%)^{b}** | **90% CI^{c}** | | **Power** | **ANOVA** |
|---|---|---|---|---|---|---|---|
| | **Test** | **Ref** | **(Test/Ref)** | **Lower** | **Upper** | | **CV%** |
| **In(Cₘₐₓ)** | 703.1707 | 1772.8716 | 39.66 | 36.36 | 43.27 | 0.9941 | 24.60 |
| **In(AUCₗₐₛₜ)** | 33655.6801 | 68872.0707 | 48.87 | 45.97 | 51.94 | 1.0000 | 17.14 |
| **ln(AUC_{inf})** | 40581.0836 | 81427.3060 | 49.84 | 46.26 | 53.70 | 0.9991 | 21.01 |

| | | | | | | | |
|---|---|---|---|---|---|---|---|
| ^{a} Geometric Mean for the Test Product (Test) and Reference Product (Ref) based on Least Squares Mean of log-transformed parameter values ^{b} Ratio(%) = Geometric Mean (Test)/Geometric Mean (Ref) ^{c} 90% Confidence Interval | | | | | | | |

**Table 61: Statistical Analysis of the Log-Transformed Systemic Exposure Parameters of Unconjugated Naloxone**

| **Dependent Variable** | **Geometric Mean^{a}** | | **Ratio (%)^{b}** | **90% CI^{c}** | | **Power** | **ANOVA** |
|---|---|---|---|---|---|---|---|
| | **Test** | **Ref** | **(Test/Ref)** | **Lower** | **Upper** | | **CV%** |
| **In(Cₘₐₓ)** | 339.8783 | 327.6977 | 103.72 | 93.78 | 114.71 | 0.9764 | 28.62 |
| **In(AUC)ₗₐₛₜ)** | 824.7608 | 868.5854 | 94.95 | 86.93 | 103.72 | 0.9931 | 24.96 |
| **ln(AUC_{inf})** | 828.9973 | 875.5051 | 94.69 | 86.79 | 103.31 | 0.9940 | 24.64 |

| | | | | | | | |
|---|---|---|---|---|---|---|---|
| ^{a} Geometric Mean for the Test Product (Test) and Reference Product (Ref) based on Least Squares Mean of log-transformed parameter values ^{b} Ratio(%) = Geometric Mean (Test)/Geometric Mean (Ref) ^{c} 90% Confidence Interval | | | | | | | |

**Table 62: Statistical Analysis of the Log-Transformed Systemic Exposure Parameters of Total Naloxone**

| **Dependent Variable** | **Geometric Mean^{a}** | | **Ratio (%)^{b}** | **90% CI^{c}** | | **Power** | **ANOVA** |
|---|---|---|---|---|---|---|---|
| | **Test** | **Ref** | **(Test/Ref)** | **Lower** | **Upper** | | **CV%** |
| **ln(Cₘₐₓ)** | 9.5951 | 20.9370 | 45.83 | 39.77 | 52.82 | 0.8295 | 41.12 |
| **ln(AUCₗₐₛₜ)** | 30.8413 | 60.7760 | 50.75 | 47.16 | 54.60 | 0.9993 | 20.61 |
| **ln(AUC_{inf})** | 32.8603 | 62.0280 | 52.98 | 49.53 | 56.67 | 0.9998 | 18.47 |

| | | | | | | | |
|---|---|---|---|---|---|---|---|
| ^{a} Geometric Mean for the Test Product (Test) and Reference Product (Ref) based on Least Squares Mean of log-transformed parameter values ^{b} Ratio(%) = Geometric Mean (Test)/Geometric Mean (Ref) ^{c} 90% Confidence Interval | | | | | | | |

**Table 63: Statistical Analysis of the Log-Transformed Partial AUCs of Buprenorphine**

| **Dependent Variable** | **Geometric Mean^{a}** | | **Ratio (%)^{b}** | **90% CI^{c}** | | **Power** | **ANOVA** |
|---|---|---|---|---|---|---|---|
| | **Test** | **Ref** | **(Test/Ref)** | **Lower** | **Upper** | | **CV**% |
| **ln(AUC₀₋₇₂)** | 42137.3401 | 47665.3869 | 88.40 | 82.59 | 94.62 | 0.9998 | 19.12 |
| **ln(AUC₀₋₉₆)** | 43841.7217 | 50179.2267 | 87.37 | 81.68 | 93.46 | 0.9998 | 18.93 |
| **ln(AUC₀₋₁₂₀)** | 44970.3912 | 51838.9927 | 86.75 | 81.12 | 92.77 | 0.9998 | 18.84 |
| **ln(AUC₀₋₁₄₄)** | 45740.0908 | 52993.1994 | 86.31 | 80.72 | 92.29 | 0.9998 | 18.82 |

| | | | | | | | |
|---|---|---|---|---|---|---|---|
| ^{a} Geometric Mean for the Test Product (Test) and Reference Product (Ref) based on Least Squares Mean of log-transformed parameter values ^{b} Ratio(%) = Geometric Mean (Test)/Geometric Mean (Ref) ^{c} 90% Confidence Interval | | | | | | | |

**Table 64: Statistical Analysis of the Log-Transformed Partial AUCs of Unconjugated Naloxone**

| **Dependent Variable** | **Geometric Mean^{a}** | | **Ratio (%)^{b}** | **90% CI^{c}** | | **Power** | ANOVA |
|---|---|---|---|---|---|---|---|
| | **Test** | **Ref** | **(Test/Ref)** | **Lower** | **Upper** | | **CV%** |
| **ln(AUC₀₋₇₂)** | 827.2680 | 874.7880 | 94.57 | 86.67 | 103.18 | 0.9939 | 24.66 |
| **ln(AUC₀₋₉₆)** | 827.9833 | 875.1413 | 94.61 | 86.71 | 103.23 | 0.9939 | 24.66 |
| **ln(AUC₀₋₁₂₀)** | 828.3676 | 875.2500 | 94.64 | 86.74 | 103.27 | 0.9939 | 24.65 |
| **ln(AUC₀₋₁₄₄)** | 828.5919 | 875.2984 | 94.66 | 86.76 | 103.29 | 0.9940 | 24.65 |

| | | | | | | | |
|---|---|---|---|---|---|---|---|
| ^{a} Geometric Mean for the Test Product (Test) and Reference Product (Ref) based on Least Squares Mean of log-transformed parameter values ^{b} Ratio(%) = Geometric Mean (Test)/Geometric Mean (Ref) ^{c} 90% Confidence Interval | | | | | | | |

### Example 10: Pharmacokinetic Data for Formulation 21

### Objective

The primary objective of this study was to compare the bioavailability of a test formulation of Buprenorphine-Naloxone Sublingual (SL) spray, 2.2 mg/0.55 mg (1 spray) to that of a single dose of Suboxone (buprenorphine and naloxone) sublingual film, 4 mg/l mg, under fasted conditions. The secondary objective was to evaluate the safety and tolerability of Buprenorphine-Naloxone SL spray.

### Study Design

This was a single-dose, open-label, randomized, two-period, two-treatment crossover study. Fifty-six healthy subjects were enrolled. Subjects who successfully completed the screening process checked into the research center the evening before first dose. Subjects who continued to meet inclusion/exclusion criteria the morning of dose were assigned a subject number, based on the order in which they successfully completed the screening process and procedures as outlined in the study protocol. Subjects were randomly assigned to a treatment sequence and received two separate single-dose administrations of study medication, one treatment per period, according to the randomization schedule. Dosing days were separated by a washout period of at least 14 days.

Subjects received each of the treatments listed below during the two treatment periods:
Treatment A: Test Product
Buprenorphine Naloxone SL spray, 2.2 mg/0.55 mg
Dose = 1 sublingual spray (total dose 2.2mg/0.55 mg)
Treatment B: Reference Product
   Suboxone^{®} (buprenorphine and naloxone) sublingual film, 4 mg/l mg
Dose = 1 × 4 mg/l mg sublingual film

### Clinical Procedures Summary

During each study period, 6 mL blood samples were obtained for buprenorphine, norbuprenorphine, and unconjugated naloxone analysis before and after each dose at selected times through 168 hours after dose administration. A total of 36 pharmacokinetic (PK) blood samples were collected from each subject for buprenorphine, norbuprenorphine, and unconjugated naloxone, 18 samples in each study period. In addition, 6 mL blood samples were obtained for total naloxone analysis before and after each dose at selected times through 72 hours after dose administration. A total of 28 PK blood samples were collected from each subject for naloxone analysis, 14 samples in each study period.

### Procedures for Collecting Samples for Pharmacokinetic Analysis

Blood samples (1 × 6 mL) for buprenorphine, norbuprenorphine, and unconjugated naloxone analysis were collected at 0 (predose), and at 5 minutes, 10 minutes, 15 minutes, 30 minutes, and 1, 2, 4, 8, 12, 24, 36, 48, 72, 96, 120, 144, and 168 hours post dose (18 time points).

Blood samples (1 × 6 mL) for total naloxone analysis were collected in Vacutainer tubes containing K₂-EDTA as a preservative at 0 (predose), and at 5 minutes, 10 minutes, 15 minutes, 30 minutes, and 1, 2, 4, 8, 12, 24, 36, 48, and 72 hours (14 time points).

### Bioanalytical Summary

Plasma samples were analyzed for buprenorphine, norbuprenorphine, unconjugated naloxone, and total naloxone by Worldwide Clinical Trials (WCT) using validated LC-MS-MS procedures. The methods were validated for ranges of 20.0 to 10,000 pg/mL for buprenorphine and norbuprenorphine and 2.00 to 1000 pg/mL for unconjugated naloxone, based on the analysis of 1.00 mL of human EDTA plasma, and 0.0500 to 50.0 ng/mL for total naloxone, based on the analysis of 0.200 mL of human EDTA plasma. Data were stored in Watson Laboratory Information Management System (LIMS; Version 7.2.0.03, Thermo Fisher Scientific). Details of the method validation and sample analysis procedure are provided in the Method Validation Report and Bioanalytical Report sections.

### Pharmacokinetic Analysis

Concentration-time data were analyzed using noncompartmental methods in Phoenix^{™} WinNonlin^{®} (Version 6.3, Pharsight Corporation). Concentration-time data that were below the limit of quantification (BLQ) were treated as zero in the data summarization and descriptive statistics. In the pharmacokinetic analysis, BLQ concentrations were treated as zero.

The following pharmacokinetic parameters were calculated: peak concentration in plasma (Cₘₐₓ), time to peak concentration (Tₘₐₓ), elimination rate constant (λ_{z}), terminal half-life (T_{1/2}), area under the concentration-time curve from time-zero to the time of the last quantifiable concentration (AUCₗₐₛₜ), area under the plasma concentration time curve from time-zero extrapolated to infinity (AUC_{inf}).

Analysis of variance (ANOVA) and the Schuirmann's two one-sided t-test procedure at the 5% significance level were applied to the log-transformed pharmacokinetic exposure parameters, Cₘₐₓ, AUCₗₐₛₜ, and AUC_{inf}. The 90% confidence interval for the ratio of the geometric means (Test/Reference) was calculated. Bioequivalence was declared if the lower and upper confidence intervals of the log-transformed parameters were within 80% to 125%.

### Results.

Data from 52 subjects who completed at least one study period were included in the pharmacokinetic analysis. Data from 50 subjects who completed both study periods were included in the statistical analysis. Mean concentration-time data are shown in Tables 65 through 68. Results of the pharmacokinetic and statistical analyses are shown below in Tables 69 through 76.

### Conclusions

Buprenorphine exposure, based on ln(AUCₗₐₛₜ) and ln(AUC_{inf}), was comparable across treatments and the 90% confidence intervals were within the accepted of 80% to 125% limits for demonstrating similar bioavailability between Buprenorphine Naloxone SL spray, 2.2 mg/0.55 mg and Suboxone sublingual film, 4 mg/l mg. Buprenorphine Cₘₐₓ was approximately 27% higher after the administration of Buprenorphine Naloxone SL spray, 2.2 mg/0.55 mg compared to that after Suboxone sublingual film, 4 mg/l mg.

Peak and overall systemic exposure to unconjugated naloxone, based on ln(Cₘₐₓ), ln(AUCₗₐₛₜ), and ln(AUC_{inf}), was approximately 31 to 66% higher after the administration of Buprenorphine Naloxone SL spray, 2.2 mg/0.55 mg compared to that after Suboxone sublingual film, 4 mg/l mg.

**Table 65: Buprenorphine Concentration-Time Data after Administration of the Test Product (Treatment A) and the Reference Product (Treatment B).**

| **Time (h)** | **Treatment A:** | | | | **Treatment B:** | | | |
|---|---|---|---|---|---|---|---|---|
| | **Test Product** | | | | **Reference Product (Suboxone)** | | | |
| | **n** | **Mean (pg/mL)** | **SD (pg/mL)** | **CV (%)** | **n** | **Mean (pg/mL)** | **SD (pg/mL)** | **CV (%)** |
| **0.00** | 50 | 0.00 | 0.00 | NC | 52 | 0.00 | 0.00 | NC |
| **0.08** | 50 | 12.9 | 42.7 | 330.40 | 52 | 0.00 | 0.00 | NC |
| **0.17** | 50 | 170 | 172 | 101.40 | 51 | 5.02 | 12.4 | 246.05 |
| **0.25** | 50 | 514 | 453 | 88.26 | 52 | 69.4 | 90.8 | 130.89 |
| **0.50** | 50 | 1360 | 809 | 59.36 | 52 | 655 | 455 | 69.47 |
| **1.00** | 50 | 2140 | 953 | 44.44 | 52 | 1470 | 633 | 43.22 |
| **2.00** | 50 | 2320 | 850 | 36.58 | 52 | 1930 | 730 | 37.89 |
| **4.00** | 50 | 1530 | 546 | 35.60 | 52 | 1310 | 539 | 41.13 |
| **8.00** | 50 | 498 | 196 | 39.33 | 52 | 494 | 191 | 38.56 |
| **12.00** | 50 | 241 | 85.0 | 35.28 | 52 | 281 | 112 | 39.66 |
| **24.00** | 50 | 120 | 45.9 | 38.13 | 52 | 158 | 66.5 | 42.21 |
| **36.00** | 49 | 80.5 | 25.4 | 31.57 | 52 | 107 | 34.6 | 32.36 |
| **48.00** | 49 | 59.8 | 19.5 | 32.53 | 52 | 76.5 | 25.5 | 33.27 |
| **72.00** | 49 | 31.7 | 15.7 | 49.54 | 52 | 45.0 | 18.5 | 41.08 |
| **96.00** | 49 | 18.1 | 15.4 | 85.22 | 52 | 27.1 | 18.1 | 66.82 |
| **120.00** | 49 | 4.48 | 10.7 | 239.47 | 52 | 12.3 | 15.4 | 125.50 |
| **144.00** | 49 | 2.19 | 7.60 | 347.35 | 52 | 4.58 | 10.5 | 229.39 |
| **168.00** | 49 | 0.580 | 4.06 | 700.00 | 52 | 1.45 | 6.10 | 420.91 |

| | | | | | | | | |
|---|---|---|---|---|---|---|---|---|
| Note: Plasma samples analyzed using a bioanalytical method with a validated range 20.0 to 10,000 pg/mL; concentrations reported in pg/mL to 3 significant figures; concentrations below limit of quantification set to zero (0.00 pg/mL) in the data summarization NC = Not calculated | | | | | | | | |

**Table 66: Norbuprenorphine Concentration-Time Data after Administration of the Test Product (Treatment A) and the Reference Product (Treatment B).**

| **Time (h)** | **Treatment A:** | | | | **Treatment B:** | | | |
|---|---|---|---|---|---|---|---|---|
| | **Test Product** | | | | **Reference Product (Suboxone)** | | | |
| | **n** | **Mean (pg/mL)** | **SD (pg/mL)** | **CV (%)** | **n** | **Mean (pg/mL)** | **SD (pg/mL)** | **CV (%)** |
| **0.00** | 50 | 0.00 | 0.00 | NC | 52 | 0.00 | 0.00 | NC |
| **0.08** | 50 | 0.00 | 0.00 | NC | 52 | 0.00 | 0.00 | NC |
| **0.17** | 50 | 4.87 | 22.6 | 463.38 | 51 | 0.613 | 4.38 | 714.14 |
| **0.25** | 50 | 33.2 | 70.6 | 212.49 | 52 | 14.4 | 39.1 | 272.22 |
| **0.50** | 50 | 119 | 149 | 124.82 | 52 | 271 | 393 | 145.18 |
| **1.00** | 50 | 193 | 155 | 80.49 | 52 | 432 | 329 | 76.08 |
| **2.00** | 50 | 217 | 117 | 53.83 | 52 | 461 | 252 | 54.53 |
| **4.00** | 50 | 196 | 89.5 | 45.56 | 52 | 364 | 168 | 46.04 |
| **8.00** | 50 | 179 | 92.1 | 51.45 | 52 | 328 | 167 | 50.84 |
| **12.00** | 50 | 164 | 83.1 | 50.69 | 52 | 305 | 159 | 52.06 |
| **24.00** | 50 | 155 | 74.4 | 48.14 | 52 | 294 | 142 | 48.10 |
| **36.00** | 49 | 130 | 56.8 | 43.66 | 52 | 237 | 97.8 | 41.33 |
| **48.00** | 49 | 106 | 44.8 | 42.23 | 52 | 188 | 79.6 | 42.44 |
| **72.00** | 49 | 70.8 | 30.5 | 43.05 | 52 | 127 | 49.7 | 39.26 |
| **96.00** | 49 | 51.5 | 28.4 | 55.22 | 52 | 90.6 | 44.6 | 49.20 |
| **120.00** | 49 | 30.6 | 24.4 | 79.65 | 52 | 58.5 | 29.6 | 50.58 |
| **144.00** | 49 | 21.2 | 22.5 | 106.17 | 52 | 39.2 | 28.7 | 73.24 |
| **168.00** | 49 | 16.2 | 20.8 | 127.83 | 52 | 29.5 | 28.0 | 94.98 |

| | | | | | | | | |
|---|---|---|---|---|---|---|---|---|
| Note: Plasma samples analyzed using a bioanalytical method with a validated range 20.0 to 10,000 pg/mL; concentrations reported in pg/mL to 3 significant figures; concentrations below limit of quantification set to zero (0.00 pg/mL) in the data summarization NC = Not calculated | | | | | | | | |

**Table 67: Unconjugated Naloxone Concentration-Time Data after Administration of the Test Product (Treatment A) and the Reference Product (Treatment B).**

| **Time (h)** | **Treatment A:** | | | | **Treatment B:** | | | |
|---|---|---|---|---|---|---|---|---|
| | **Test Product** | | | | **Reference Product (Suboxone)** | | | |
| | **n** | **Mean (pg/mL)** | **SD (pg/mL)** | **CV (%)** | **n** | **Mean (pg/mL)** | **SD (pg/mL)** | **CV (%)** |
| **0.00** | 50 | 0.00 | 0.00 | NC | 52 | 0.00 | 0.00 | NC |
| **0.08** | 50 | 22.6 | 28.7 | 127.14 | 52 | 0.141 | 0.710 | 505.16 |
| **0.17** | 50 | 69.6 | 48.1 | 69.17 | 51 | 8.12 | 11.0 | 135.57 |
| **0.25** | 50 | 115 | 80.1 | 69.56 | 52 | 27.7 | 32.2 | 116.37 |
| **0.50** | 50 | 140 | 71.9 | 51.43 | 52 | 75.1 | 49.2 | 65.49 |
| **1.00** | 50 | 112 | 50.5 | 44.89 | 52 | 82.3 | 37.7 | 45.84 |
| **2.00** | 50 | 65.3 | 34.4 | 52.62 | 52 | 52.4 | 20.4 | 38.88 |
| **4.00** | 50 | 21.8 | 14.7 | 67.22 | 52 | 18.4 | 10.1 | 54.68 |
| **8.00** | 50 | 3.01 | 2.55 | 84.83 | 52 | 4.09 | 3.42 | 83.71 |
| **12.00** | 50 | 0.480 | 1.23 | 257.25 | 52 | 1.46 | 2.43 | 166.08 |
| **24.00** | 50 | 0.0598 | 0.423 | 707.11 | 52 | 0.297 | 0.944 | 318.00 |
| **36.00** | 49 | 0.00 | 0.00 | NC | 52 | 0.00 | 0.00 | NC |
| **48.00** | 49 | 0.00 | 0.00 | NC | 52 | 0.00 | 0.00 | NC |
| **72.00** | 49 | 0.00 | 0.00 | NC | 52 | 0.00 | 0.00 | NC |
| **96.00** | 49 | 0.00 | 0.00 | NC | 52 | 0.00 | 0.00 | NC |
| **120.00** | 49 | 0.00 | 0.00 | NC | 52 | 0.00 | 0.00 | NC |
| **144.00** | 49 | 0.00 | 0.00 | NC | 52 | 0.00 | 0.00 | NC |
| **168.00** | 49 | 0.00 | 0.00 | NC | 52 | 0.00 | 0.00 | NC |

| | | | | | | | | |
|---|---|---|---|---|---|---|---|---|
| Note: Plasma samples analyzed using a bioanalytical method with a validated range 2.00 to 1000 pg/mL; concentrations reported in pg/mL to 3 significant figures; concentrations below limit of quantification set to zero (0.00 pg/mL) in the data summarization NC = Not calculated | | | | | | | | |

**Table 68: Total Naloxone Concentration-Time Data after Administration of the Test Product (Treatment A) and the Reference Product (Treatment B).**

| **Time (h)** | **Treatment A:** | | | | **Treatment B:** | | | |
|---|---|---|---|---|---|---|---|---|
| | **Test Product** | | | | **Reference Product (Suboxone)** | | | |
| | **n** | **Mean (ng/mL)** | **SD (ng/mL)** | **CV (%)** | **n** | **Mean (ng/mL)** | **SD (ng/mL)** | **CV (%)** |
| **0.00** | 50 | 0.00 | 0.00 | NC | 52 | 0.00 | 0.00 | NC |
| **0.08** | 50 | 0.0538 | 0.116 | 216.21 | 52 | 0.00453 | 0.0198 | 437.39 |
| **0.17** | 50 | 0.486 | 0.756 | 155.58 | 51 | 0.105 | 0.307 | 292.49 |
| **0.25** | 50 | 1.36 | 1.60 | 117.10 | 52 | 1.02 | 1.54 | 150.08 |
| **0.50** | 50 | 2.69 | 2.32 | 86.33 | 52 | 7.95 | 6.80 | 85.51 |
| **1.00** | 50 | 3.22 | 2.34 | 72.61 | 52 | 6.28 | 3.58 | 57.03 |
| **2.00** | 50 | 1.74 | 0.983 | 56.49 | 52 | 3.50 | 1.71 | 48.75 |
| **4.00** | 50 | 0.658 | 0.468 | 71.21 | 52 | 1.21 | 0.902 | 74.26 |
| **8.00** | 50 | 0.321 | 0.146 | 45.65 | 52 | 0.570 | 0.290 | 50.96 |
| **12.00** | 50 | 0.198 | 0.0980 | 49.49 | 52 | 0.372 | 0.187 | 50.21 |
| **24.00** | 50 | 0.0679 | 0.0496 | 73.01 | 52 | 0.124 | 0.0642 | 51.62 |
| **36.00** | 49 | 0.00129 | 0.00904 | 700.00 | 52 | 0.0116 | 0.0304 | 261.88 |
| **48.00** | 49 | 0.00 | 0.00 | NC | 52 | 0.00 | 0.00 | NC |
| **72.00** | 49 | 0.00 | 0.00 | NC | 52 | 0.00 | 0.00 | NC |

| | | | | | | | | |
|---|---|---|---|---|---|---|---|---|
| Note: Plasma samples analyzed using a bioanalytical method with a validated range 0.0500 to 50.0 ng/mL; concentrations reported in ng/mL to 3 significant figures; concentrations below limit of quantification set to zero (0.00 ng/mL) in the data summarization NC = Not calculated | | | | | | | | |

**Table 69: Pharmacokinetic Parameters of Buprenorphine**

| **Parameter** | **Treatment A:** | | | | **Treatment B:** | | | |
|---|---|---|---|---|---|---|---|---|
| | **Test Product** | | | | **Reference Product (Suboxone)** | | | |
| | **n** | **Mean** | **SD** | **CV%** | **n** | **Mean** | **SD** | **CV%** |
| **Tₘₐₓ (h)** | 50 | 1.68 | 0.73 | 43.26 | 52 | 1.98 | 0.72 | 36.38 |
| **Cₘₐₓ (pg/mL)** | 50 | 2470 | 850 | 34.35 | 52 | 1990 | 703 | 35.43 |
| **AUCₗₐₛₜ (h*pg/mL)** | 50 | 18010 | 6118 | 33.97 | 52 | 18240 | 5820 | 31.91 |
| **AUC_{inf} (h*pg/mL)** | 50 | 19320 | 6190 | 32.04 | 52 | 19590 | 6018 | 30.72 |
| **AUC_{Extrap} (%)** | 50 | 7.39 | 3.84 | 51.88 | 52 | 7.23 | 2.65 | 36.72 |
| **λ_{z} (h⁻¹)** | 50 | 0.0244 | 0.0130 | 53.20 | 52 | 0.0217 | 0.0078 | 35.71 |
| **T_{1/2} (h)** | 50 | 33.99 | 14.07 | 41.40 | 52 | 35.59 | 11.28 | 31.69 |
| **Tₗₐₛₜ (h)** | 50 | 89.28 | 27.87 | 31.22 | 52 | 105.23 | 28.97 | 27.53 |
| **Cₗₐₛₜ (pg/mL)** | 50 | 28.2 | 11.0 | 38.96 | 52 | 26.4 | 5.98 | 22.65 |

| | | | | | | | | |
|---|---|---|---|---|---|---|---|---|
| Note: Full precision data used in pharmacokinetic analysis | | | | | | | | |

**Table 70: Pharmacokinetic Parameters of Norbuprenorphine**

| **Parameter** | **Treatment A:** | | | | **Treatment B:** | | | |
|---|---|---|---|---|---|---|---|---|
| | **Test Product** | | | | **Reference Product (Suboxone)** | | | |
| | **n** | **Mean** | **SD** | **CV%** | **n** | **Mean** | **SD** | |
| **Tₘₐₓ (h)** | 50 | 5.54 | 8.06 | 145.49 | 52 | 4.00 | 5.62 | 140.60 |
| **Cₘₐₓ (pg/mL)** | 50 | 265 | 162 | 61.11 | 52 | 566 | 350 | 61.76 |
| **AUCₗₐₛₜ (h*pg/mL)** | 50 | 12360 | 5387 | 43.57 | 52 | 23270 | 9030 | 38.80 |
| **AUC_{inf} (h*pg/mL)** | 50 | 15370 | 6778 | 44.09 | 52 | 26980 | 11550 | 42.82 |
| **AUC_{Extrap} (%)** | 50 | 19.24 | 12.97 | 67.42 | 52 | 12.48 | 11.68 | 93.57 |
| **λ_{z} (h⁻¹)** | 50 | 0.0165 | 0.0095 | 57.42 | 52 | 0.0168 | 0.0072 | 42.94 |
| **T_{1/2} (h)** | 50 | 56.34 | 39.94 | 70.89 | 52 | 53.41 | 42.88 | 80.29 |
| **Tₗₐₛₜ (h)** | 50 | 131.52 | 38.25 | 29.09 | 52 | 152.77 | 23.78 | 15.56 |
| **Cₗₐₛₜ (pg/mL)** | 50 | 34.0 | 13.8 | 40.44 | 52 | 39.0 | 20.0 | 51.24 |

| | | | | | | | | |
|---|---|---|---|---|---|---|---|---|
| Note: Full precision data used in pharmacokinetic analysis | | | | | | | | |

**Table 71: Pharmacokinetic Parameters of Unconjugated Naloxone**

| **Parameter** | **Treatment A:** | | | | **Treatment B:** | | | |
|---|---|---|---|---|---|---|---|---|
| | **Test Product** | | | | **Reference Product (Suboxone)** | | | |
| | **n** | **Mean** | **SD** | **CV%** | **n** | **Mean** | **SD** | **CV%** |
| **Tₘₐₓ (h)** | 50 | 0.54 | 0.26 | 47.66 | 52 | 0.95 | 0.45 | 46.82 |
| **Cₘₐₓ (pg/mL)** | 50 | 153 | 78.4 | 51.37 | 52 | 89.8 | 43.9 | 48.83 |
| **AUCₗₐₛₜ (h*pg/mL)** | 50 | 310.2 | 156.8 | 50.57 | 52 | 232.6 | 105.0 | 45.16 |
| **AUC_{inf} (h*pg/mL)** | 50 | 320.6 | 158.3 | 49.37 | 44 | 262.0 | 110.1 | 42.04 |
| **AUC_{Extrap} (%)** | 50 | 4.09 | 3.98 | 97.30 | 44 | 4.86 | 2.83 | 58.26 |
| **λ_{z} (h^{-l})** | 50 | 0.4972 | 0.1191 | 23.95 | 44 | 0.3643 | 0.1447 | 39.72 |
| **T_{1/2} (h)** | 50 | 1.58 | 1.06 | 67.25 | 44 | 2.60 | 2.28 | 87.94 |
| **Tₗₐₛₜ (h)** | 50 | 7.92 | 3.38 | 42.67 | 52 | 10.15 | 5.16 | 50.83 |
| **Cₗₐₛₜ (pg/mL)** | 50 | 5.28 | 4.51 | 85.35 | 52 | 4.16 | 2.52 | 60.67 |

| | | | | | | | | |
|---|---|---|---|---|---|---|---|---|
| Note: Full precision data used in pharmacokinetic analysis | | | | | | | | |

**Table 72: Pharmacokinetic Parameters of Total Naloxone**

| **Parameter** | **Treatment A:** | | | | **Treatment B:** | | | |
|---|---|---|---|---|---|---|---|---|
| | **Test Product** | | | | **Reference Product (Suboxone)** | | | |
| | **n** | **Mean** | **SD** | **CV%** | **n** | **Mean** | **SD** | **CV%** |
| **Tₘₐₓ (h)** | 50 | 1.17 | 1.26 | 108.00 | 52 | 1.02 | 0.70 | 68.67 |
| **Cₘₐₓ (ng/mL)** | 50 | 4.26 | 2.52 | 59.05 | 52 | 9.95 | 5.47 | 54.92 |
| **AUCₗₐₛₜ (h*ng/mL)** | 50 | 10.68 | 3.908 | 36.60 | 52 | 21.34 | 6.554 | 30.72 |
| **AUC_{inf} (h*ng/mL)** | 49 | 11.87 | 3.903 | 32.89 | 52 | 22.70 | 6.714 | 29.58 |
| **AUC_{Extrap} (%)** | 49 | 9.54 | 7.78 | 81.57 | 52 | 6.24 | 3.59 | 57.52 |
| **λ_{z} (h⁻¹)** | 49 | 0.1161 | 0.0579 | 49.87 | 52 | 0.1066 | 0.0372 | 34.84 |
| **T_{1/2} (h)** | 49 | 7.21 | 3.33 | 46.21 | 52 | 7.35 | 2.81 | 38.28 |
| **Tₗₐₛₜ (h)** | 50 | 21.04 | 5.87 | 27.91 | 52 | 24.69 | 5.53 | 22.39 |
| **Cₗₐₛₜ (ng/mL)** | 50 | 0.102 | 0.0428 | 42.00 | 52 | 0.136 | 0.104 | 76.93 |

| | | | | | | | | |
|---|---|---|---|---|---|---|---|---|
| Note: Full precision data used in pharmacokinetic analysis | | | | | | | | |

**Table 73: Statistical Analysis of the Log-Transformed Systemic Exposure Parameters of Buprenorphine**

| **Dependent** | **Geometric Mean^{a}** | | **Ratio (%)^{b}** | **90% CI^{c}** | | **Power** | **ANOVA CV%** |
|---|---|---|---|---|---|---|---|
| **Variable** | **Test** | **Ref** | **(Test/Ref)** | **Lower** | **Upper** | | |
| **ln(Cₘₐₓ)** | 2334.8796 | 1842.7190 | 126.71 | 114.98 | 139.63 | 0.9827 | 29.55 |
| **ln(AUCₗₐₛₜ)** | 17009.6037 | 17098.2817 | 99.48 | 91.06 | 108.69 | 0.9930 | 26.82 |
| **In(AUC_{inf})** | 18379.2372 | 18433.5928 | 99.71 | 91.61 | 108.52 | 0.9957 | 25.64 |

| | | | | | | | |
|---|---|---|---|---|---|---|---|
| ^{a} Geometric Mean for the Test Product (Test) and Reference Product (Ref) based on Least Squares Mean of log-transformed parameter values ^{b} Ratio(%) = Geometric Mean (Test)/Geometric Mean (Ref) ^{c} 90% Confidence Interval | | | | | | | |

**Table 74: Statistical Analysis of the Log-Transformed Systemic Exposure Parameters of Norbuprenorphine**

| **Dependent** | **Geometric Mean^{a}** | | **Ratio (%)^{b}** | **90% CI^{c}** | | **Power** | **ANOVA CV%** |
|---|---|---|---|---|---|---|---|
| **Variable** | **Test** | **Ref** | **(Test/Ref)** | **Lower** | **Upper** | | |
| **ln(Cₘₐₓ)** | 228.7018 | 489.3838 | 46.73 | 43.28 | 50.47 | 0.9988 | 23.19 |
| **ln(AUCₗₐₛₜ)** | 11116.0926 | 21710.7037 | 51.20 | 47.09 | 55.67 | 0.9963 | 25.34 |
| **In(AUC_{inf})** | 13986.5409 | 24965.8998 | 56.02 | 51.65 | 60.77 | 0.9974 | 24.59 |

| | | | | | | | |
|---|---|---|---|---|---|---|---|
| ^{a} Geometric Mean for the Test Product (Test) and Reference Product (Ref) based on Least Squares Mean of log-transformed parameter values ^{b} Ratio(%) = Geometric Mean (Test)/Geometric Mean (Ref) ^{c} 90% Confidence Interval | | | | | | | |

**Table 75: Statistical Analysis of the Log-Transformed Systemic Exposure Parameters of Unconjugated Naloxone**

| **Dependent** | **Geometric Mean^{a}** | | **Ratio (%)^{b}** | **90% CI^{c}** | | **Power** | **ANOVA CV%** |
|---|---|---|---|---|---|---|---|
| **Variable** | **Test** | **Ref** | **(Test/Ref)** | **Lower** | **Upper** | | |
| **ln(Cₘₐₓ)** | 132.4558 | 79.8936 | 165.79 | 146.96 | 187.03 | 0.9200 | 37.10 |
| **ln(AUCₗₐₛₜ)** | 275.6491 | 210.1213 | 131.19 | 117.86 | 146.02 | 0.9622 | 32.75 |
| **ln(AUC_{inf})** | 287.6305 | 218.5298 | 131.62 | 118.45 | 146.26 | 0.9663 | 29.60 |

| | | | | | | | |
|---|---|---|---|---|---|---|---|
| ^{a}Geometric Mean for the Test Product (Test) and Reference Product (Ref) based on Least Squares Mean of log-transformed parameter values ^{b} Ratio(%) = Geometric Mean (Test)/Geometric Mean (Ref) ^{c} 90% Confidence Interval | | | | | | | |

**Table 76: Statistical Analysis of the Log-Transformed Systemic Exposure Parameters of Total Naloxone**

| **Dependent** | **Geometric Mean^{a}** | | **Ratio (%)^{b}** | **90% CI^{c}** | | **Power** | **ANOVA CV%** |
|---|---|---|---|---|---|---|---|
| **Variable** | **Test** | **Ref** | **(Test/Ref)** | **Lower** | **Upper** | | |
| **In(Cₘₐₓ)** | 3.4477 | 8.5476 | 40.34 | 34.96 | 46.53 | 0.8245 | 44.57 |
| **ln(AUCₗₐₛₜ)** | 9.8049 | 20.6392 | 47.51 | 44.24 | 51.01 | 0.9996 | 21.45 |
| **ln(AUC_{inf})** | 11.1098 | 22.0499 | 50.39 | 47.23 | 53.75 | 0.9999 | 19.22 |

| | | | | | | | |
|---|---|---|---|---|---|---|---|
| ^{a} Geometric Mean for the Test Product (Test) and Reference Product (Ref) based on Least Squares Mean of log-transformed parameter values ^{b} Ratio(%) = Geometric Mean (Test)/Geometric Mean (Ref) ^{c} 90% Confidence Interval | | | | | | | |

## Claims

1. A liquid formulation for use in treating acute pain, wherein the formulation is a sublingual spray formulation comprising:
buprenorphine, or a pharmaceutically acceptable salt thereof, in an amount from 0.1625% to 0.65% w/w;
water as a solvent in an amount from 38% to 40% w/w;
a cosolvent consisting of a mixture of ethanol in an amount of 55% w/w and propylene glycol in an amount of about 5% w/w;
an antioxidant consisting of a mixture of butylated hydroxyanisole (BHA) in an amount of about 0.01% w/w and butylated hydroxytoluene (BHT) in an amount of about 0.005% w/w; and
menthol in an amount of about 0.05% w/w,
wherein the % w/w is of the total formulation.

2. The liquid formulation for use
of claim 1 wherein the formulation is a sublingual spray
formulation and is capable of producing a droplet size distribution wherein greater than 98% of the composition particles are greater than 10 microns in diameter during administration; or wherein
the formulation is a sublingual spray formulation and is capable of producing a droplet size distribution wherein:
the mean Dv(10) is from 10 to 40 microns during administration;
the mean Dv(50) is from 30 to 80 microns during administration; and
the mean Dv(90) is from 80 to 200 microns during administration; or wherein
the formulation is a sublingual spray formulation and is capable of producing a spray plume that has an ovality ratio of from 1.1 to 2.4.

3. The liquid formulation for use of claim 1 wherein the formulation is capable of producing a spray plume width that is from 25 to 45 millimeters during administration and a spray plume angle that is from 30 to 55 degrees during administration; or wherein
the formulation is capable of producing a D(4,3) of 50 to 95 microns; or wherein
the formulation the formulation is a sublingual spray formulation that is capable of producing a droplet size distribution wherein the Cₘₐₓ (ng/mL) of buprenorphine is from 0.6 to 1.5; or wherein
the formulation is a sublingual spray formulation that is capable of producing a droplet size distribution wherein the Tₘₐₓ of buprenorphine is from 1.5 to 1.9 hours following administration.

## Patentansprüche

1. Flüssige Formulierung zur Verwendung beim Behandeln akuter Schmerzen, wobei die Formulierung eine sublinguale Sprühformulierung ist, umfassend:
Buprenorphin oder ein pharmazeutisch annehmbares Salz davon in einer Menge von 0,1625 bis 0,65 Gewichtsprozent;
Wasser als Lösungsmittel in einer Menge von 38 bis 40 Gewichtsprozent;
ein Co-Lösungsmittel, bestehend aus einem Gemisch aus Ethanol in einer Menge von 55 Gewichtsprozent und Propylenglykol in einer Menge von etwa 5 Gewichtsprozent;
ein Antioxidationsmittel, bestehend aus einem Gemisch aus butyliertem Hydroxyanisol (BHA) in einer Menge von etwa 0,01 Gewichtsprozent und butyliertem Hydroxytoluol (BHT) in einer Menge von etwa 0,005 Gewichtsprozent; und
Menthol in einer Menge von etwa 0,05 Gewichtsprozent, wobei sich die Gewichtsprozent auf die gesamte Formulierung beziehen.

2. Flüssige Formulierung zur Verwendung nach Anspruch 1, wobei die Formulierung eine sublinguale Sprühformulierung ist und in der Lage ist, eine Tröpfchengrößenverteilung zu erzeugen, wobei mehr als 98 % der Teilchen der Zusammensetzung während einer Verabreichung einen Durchmesser von mehr als 10 Mikrometern aufweisen; oder wobei
die Formulierung eine sublinguale Sprühformulierung ist und eine Tröpfchengrößenverteilung erzeugen kann, wobei:
der mittlere Dv(10) während einer Verabreichung von 10 bis 40 Mikrometer ist;
der mittlere Dv(50) während einer Verabreichung von 30 bis 80 Mikrometer ist; und
der mittlere Dv(90) während einer Verabreichung von 80 bis 200 Mikrometer ist; oder wobei
die Formulierung eine sublinguale Sprühformulierung ist und ist in der Lage, eine Sprühfahne zu erzeugen, die ein Ovalitätsverhältnis von 1,1 bis 2,4 aufweist.

3. Flüssige Formulierung zur Verwendung nach Anspruch 1, wobei die Formulierung in der Lage ist, eine Sprühfahnenbreite, die während einer Verabreichung von 25 bis 45 Millimeter ist, und einen Sprühfahnenwinkel, der während einer Verabreichung von 30 bis 55 Grad ist, zu erzeugen; oder wobei
die Formulierung in der Lage ist, einen D(4,3) von 50 bis 95 Mikrometer zu erzeugen; oder wobei
die Formulierung die Formulierung eine sublinguale Sprühformulierung ist, die in der Lage ist, eine Tröpfchengrößenverteilung zu erzeugen, wobei die Cₘₐₓ (ng/ml) von Buprenorphin von 0,6 bis 1,5 ist; oder wobei
die Formulierung eine sublinguale Sprühformulierung ist, die in der Lage ist, eine Tröpfchengrößenverteilung zu erzeugen, wobei die Tₘₐₓ von Buprenorphin von 1,5 bis 1,9 Stunden nach einer Verabreichung ist.

## Revendications

1. Formulation liquide destinée à être utilisée dans le traitement de la douleur aiguë, dans laquelle la formulation est une formulation de pulvérisation sublinguale comprenant :
de la buprénorphine, ou un de ses sels pharmaceutiquement acceptables, en une quantité allant de 0,1625 % à 0,65 % p/p ;
de l'eau comme solvant en une quantité allant de 38 % à 40 % p/p ;
un cosolvant constitué d'un mélange d'éthanol en une quantité de 55 % p/p et de propylène glycol en une quantité d'environ 5 % p/p ;
un antioxydant constitué d'un mélange d'hydroxyanisole butylé (BHA) en une quantité d'environ 0,01 % p/p et d'hydroxytoluène butylé (BHT) en une quantité d'environ 0,005 % p/p ; et
du menthol en une quantité d'environ 0,05 % p/p, le pourcentage p/p étant celui de la formulation totale.

2. Formulation liquide destinée à être utilisée selon la revendication 1, dans laquelle la formulation est une formulation de pulvérisation sublinguale et est capable de produire une distribution de taille de gouttelettes dans laquelle plus de 98 % des particules de la composition ont un diamètre supérieur à 10 microns lors de l'administration ; ou dans laquelle la formulation est une formulation de pulvérisation sublinguale et est capable de produire une distribution de taille de gouttelettes dans laquelle :
la Dv(10) moyenne est comprise entre 10 et 40 microns pendant l'administration ;
la Dv(50) moyenne est comprise entre 30 et 80 microns pendant l'administration ; et
la Dv(90) moyenne est comprise entre 80 et 200 microns pendant l'administration ; ou dans laquelle
la formulation est une formulation de pulvérisation sublinguale et est capable de produire un panache de pulvérisation dont le rapport d'ovalisation est compris entre 1,1 et 2,4.

3. Formulation liquide destinée à être utilisée selon la revendication 1, dans laquelle la formulation est capable de produire une largeur de panache de pulvérisation comprise entre 25 et 45 millimètres pendant l'administration et un angle de panache de pulvérisation compris entre 30 et 55 degrés pendant l'administration ; ou dans laquelle
la formulation est capable de produire une D(4,3) comprise entre 50 et 95 microns ; ou dans laquelle
la formulation la formulation est une formulation de pulvérisation sublinguale capable de produire une distribution de taille de gouttelettes dans laquelle la Cₘₐₓ (ng/mL) de la buprénorphine est comprise entre 0,6 et 1,5 ; ou dans laquelle
la formulation est une formulation de spray sublingual capable de produire une distribution de la taille de gouttelettes dans laquelle la Tₘₐₓ de la buprénorphine est comprise entre 1,5 et 1,9 heures après l'administration.
